# EUROPEAN PATENT SPECIFICATION

(11) **EP 3 067 419 B1**
(45) Date of publication and mention of the grant of the patent: **17.03.2021**
(21) Application number: 14860703.9
(22) Date of filing: 30.10.2014
(51) Int. Cl.: C12N 15/09, A01K 67/027, C12N 1/15, C12N 1/19, C12N 1/21, C12N 5/10, C12N 15/90, C12N 15/85, C12N 15/10

(54) **VECTOR FOR NUCLEIC ACID INSERTION**
VEKTOR FÜR NUKLEINSÄUREINSERTION
VECTEUR POUR L'INSERTION D'UN ACIDE NUCLÉIQUE

(30) Priority: 06.11.2013 JP 2013230349
(43) Date of publication of application: 14.09.2016
(73) Proprietor: Hiroshima University, Higashihiroshima-shi Hiroshima 739-8511 (JP)
(72) Inventor: YAMAMOTO, Takashi, Higashihiroshima-shi Hiroshima 739-8526 (JP); SUZUKI, Kenichi, Higashihiroshima-shi Hiroshima 739-8526 (JP); SAKUMA, Tetsushi, Higashihiroshima-shi Hiroshima 739-8526 (JP); SAKANE, Yuto, Higashihiroshima-shi Hiroshima 739-8526 (JP)
(74) Representative: Boult Wade Tennant LLP
(86) International application number: PCT/JP2014/079515
(87) International publication number: WO 2015/068785

(56) References cited:
- WO-A1-2011/011767
- JP-A- 2013 500 018
- DIRK HOCKEMEYER ET AL: "Genetic engineering of human pluripotent cells using TALE nucleases", NATURE BIOTECHNOLOGY, vol. 29, no. 8, 7 July 2011 (2011-07-07), pages 731-734, XP055265362, US ISSN: 1087-0156, DOI: 10.1038/nbt.1927 & Dirk Hockemeyer et al.: "SUPPLEMENTARY INFORMATION EXPERIMENTAL PROCEDURES", , 7 July 2011 (2011-07-07), XP55390530, Retrieved from the Internet: URL:https://www.ncbi.nlm.nih.gov/pmc/artic les/PMC3152587/bin/NIHMS307793-supplement- 1.pdf [retrieved on 2017-07-13]
- DIRK HOCKEMEYER ET AL: "Efficient targeting of expressed and silent genes in human ESCs and iPSCs using zinc-finger nucleases", NATURE BIOTECHNOLOGY, vol. 27, no. 9, 13 August 2009 (2009-08-13), pages 851-857, XP55390559, US ISSN: 1087-0156, DOI: 10.1038/nbt.1562
- SANDRA CRISTEA ET AL: "In vivo cleavage of transgene donors promotes nuclease-mediated targeted integration", BIOTECHNOLOGY AND BIOENGINEERING, vol. 110, no. 3, 1 March 2013 (2013-03-01) , pages 871-880, XP055076901, DOI: 10.1002/bit.24733
- M. MARESCA ET AL: "Obligate Ligation-Gated Recombination (ObLiGaRe): Custom-designed nuclease-mediated targeted integration through nonhomologous end joining", GENOME RESEARCH, vol. 23, no. 3, 14 November 2012 (2012-11-14), pages 539-546, XP055077484, ISSN: 1088-9051, DOI: 10.1101/gr.145441.112
- S. J. ORLANDO ET AL: "Zinc-finger nuclease-driven targeted integration into mammalian genomes using donors with limited chromosomal homology", NUCLEIC ACIDS RESEARCH, vol. 38, no. 15, 8 June 2010 (2010-06-08), pages e152-e152, XP055076783, ISSN: 0305-1048, DOI: 10.1093/nar/gkq512
- H. OCHIAI ET AL: "Zinc-finger nuclease-mediated targeted insertion of reporter genes for quantitative imaging of gene expression in sea urchin embryos", PROCEEDINGS NATIONAL ACADEMY OF SCIENCES PNAS, vol. 109, no. 27, 18 June 2012 (2012-06-18), pages 10915-10920, XP055342773, US ISSN: 0027-8424, DOI: 10.1073/pnas.1202768109
- THOMAS O. AUER ET AL: "CRISPR/Cas9 and TALEN-mediated knock-in approaches in zebrafish", METHODS, vol. 69, no. 2, 1 April 2014 (2014-04-01), pages 142-150, XP055277728, US ISSN: 1046-2023, DOI: 10.1016/j.ymeth.2014.03.027
- TETSUSHI SAKUMA ET AL: "MMEJ-assisted gene knock-in using TALENs and CRISPR-Cas9 with the PITCh systems", NATURE PROTOCOLS, vol. 11, no. 1, 17 December 2015 (2015-12-17), pages 118-133, XP55388413, GB ISSN: 1754-2189, DOI: 10.1038/nprot.2015.140
- OCHIAI H. ET AL.: 'Zinc-finger nuclease-mediated targeted insertion of reporter genes for quantitative imaging of gene expression in sea urchin embryos' PNAS vol. 109, no. 27, pages 10915 - 10920, XP055342773
- HIROSHI OCHIAI ET AL.: 'TALE nuclease (TALEN) o Mochiita Baiyo Saibo ni Okeru Genome Henshu' EXPERIMENTAL MEDICINE vol. 31, no. 1, January 2013, pages 95 - 100, XP008183323
- MALI P. ET AL.: 'RNA-Guided Human Genome Engineering via Cas9' SCIENCE vol. 339, February 2013, pages 823 - 826, XP055337932
- MARESCA M. ET AL.: 'Obligate Ligation-Gated Recombination (ObLiGaRe):Custom-designed nuclease-mediated targeted integration through nonhomologous end joining' GENOME RESEARCH vol. 23, March 2013, pages 539 - 546, XP055077484
- CRISTEA S. ET AL.: 'In vivo Cleavage of Transgene Donors Promotes Nuclease-Mediated Targeted Integration' BIOTECHNOLOGY AND BIOENGINEERING vol. 110, no. 3, March 2013, pages 871 - 880, XP055076901
- TAKASHI YAMAMOTO ET AL.: '1. Genome editing with programmable site-specific nucleases' VIRUS vol. 64, no. 1, June 2014, pages 75 - 82, XP008183223
- NAKADE S. ET AL.: 'Microhomology-mediated end- joining-dependent integration of donor DNA in cells and animals using TALENs and CRISPR/Cas9' NATURE COMMUNICATIONS vol. 5, no. 5560, 20 November 2014, pages 1 - 8, XP055342780

### SEQUENCE LISTING

<110> HIROSHIMA UNIVERSITY
<120> A vector for inserting a nucleic acid
<130> 672130
<160> 23
<170> PatentIn version 3.5
<210> 1
   <211> 8465
   <212> DNA
   <213> Artificial Sequence
<220>
   <223> Left TALEN
<220>
   <221> CDS
   <222> (5131)..(8433)
<400> 1 tcgactgtgc cttctagttg cc 8465
<210> 2
   <211> 1100
   <212> PRT
   <213> Artificial Sequence
<220>
   <223> Synthetic Construct
<400> 2
   Met Ala Ser Ser Pro Pro Lys Lys Lys Arg Lys Val Ala Ala Ala Asp
   1               5                   10                  15
   Tyr Lys Asp Asp Asp Asp Lys Ser Trp Lys Asp Ala Ser Gly Trp Ser
               20                  25                  30
   Arg Met His Ala Ala Pro Arg Arg Arg Ala Ala Gln Pro Ser Asp Ala
           35                  40                  45
   Ser Pro Ala Ala Gln Val Asp Leu Arg Thr Leu Gly Tyr Ser Gln Gln
       50                  55                  60
   Gln Gln Glu Lys Ile Lys Pro Lys Val Arg Ser Thr Val Ala Gln His
   65                  70                  75                  80
   His Glu Ala Leu Val Gly His Gly Phe Thr His Ala His Ile Val Ala
                   85                  90                  95
   Leu Ser Gln His Pro Ala Ala Leu Gly Thr Val Ala Val Thr Tyr Gln
               100                 105                 110
   His Ile Ile Thr Ala Leu Pro Glu Ala Thr His Glu Asp Ile Val Gly
           115                 120                 125
   Val Gly Lys Gln Trp Ser Gly Ala Arg Ala Leu Glu Ala Leu Leu Thr
       130                 135                 140
   Asp Ala Gly Glu Leu Arg Gly Pro Pro Leu Gln Leu Asp Thr Gly Gln
   145                 150                 155                 160
   Leu Val Lys Ile Ala Lys Arg Gly Gly Val Thr Ala Met Glu Ala Val
                   165                 170                 175
   His Ala Ser Arg Asn Ala Leu Thr Gly Ala Pro Leu Asn Leu Thr Pro
               180                 185                 190
   Asp Gln Val Val Ala Ile Ala Ser Asn Ile Gly Gly Lys Gln Ala Leu
           195                 200                 205
   Glu Thr Val Gln Arg Leu Leu Pro Val Leu Cys Gln Asp His Gly Leu
       210                 215                 220
   Thr Pro Glu Gln Val Val Ala Ile Ala Ser His Asp Gly Gly Lys Gln
   225                 230                 235                 240
   Ala Leu Glu Thr Val Gln Arg Leu Leu Pro Val Leu Cys Gln Ala His
                   245                 250                 255
   Gly Leu Thr Pro Asp Gln Val Val Ala Ile Ala Ser Asn Gly Gly Gly
               260                 265                 270
   Lys Gln Ala Leu Glu Thr Val Gln Arg Leu Leu Pro Val Leu Cys Gln
           275                 280                 285
   Ala His Gly Leu Thr Pro Ala Gln Val Val Ala Ile Ala Ser Asn Gly
       290                 295                 300
   Gly Gly Lys Gln Ala Leu Glu Thr Val Gln Arg Leu Leu Pro Val Leu
   305                 310                 315                 320
   Cys Gln Asp His Gly Leu Thr Pro Asp Gln Val Val Ala Ile Ala Ser
                   325                 330                 335
   His Asp Gly Gly Lys Gln Ala Leu Glu Thr Val Gln Arg Leu Leu Pro
               340                 345                 350
   Val Leu Cys Gln Asp His Gly Leu Thr Pro Glu Gln Val Val Ala Ile
           355                 360                 365
   Ala Ser Asn Gly Gly Gly Lys Gln Ala Leu Glu Thr Val Gln Arg Leu
       370                 375                 380
   Leu Pro Val Leu Cys Gln Ala His Gly Leu Thr Pro Asp Gln Val Val
   385                 390                 395                 400
   Ala Ile Ala Ser Asn Gly Gly Gly Lys Gln Ala Leu Glu Thr Val Gln
                   405                 410                 415
   Arg Leu Leu Pro Val Leu Cys Gln Ala His Gly Leu Thr Pro Ala Gln
               420                 425                 430
   Val Val Ala Ile Ala Ser Asn Asn Gly Gly Lys Gln Ala Leu Glu Thr
           435                 440                 445
   Val Gln Arg Leu Leu Pro Val Leu Cys Gln Asp His Gly Leu Thr Pro
       450                 455                 460
   Asp Gln Val Val Ala Ile Ala Ser His Asp Gly Gly Lys Gln Ala Leu
   465                 470                 475                 480
   Glu Thr Val Gln Arg Leu Leu Pro Val Leu Cys Gln Asp His Gly Leu
                   485                 490                 495
   Thr Pro Glu Gln Val Val Ala Ile Ala Ser Asn Gly Gly Gly Lys Gln
               500                 505                 510
   Ala Leu Glu Thr Val Gln Arg Leu Leu Pro Val Leu Cys Gln Ala His
           515                 520                 525
   Gly Leu Thr Pro Asp Gln Val Val Ala Ile Ala Ser Asn Asn Gly Gly
       530                 535                 540
   Lys Gln Ala Leu Glu Thr Val Gln Arg Leu Leu Pro Val Leu Cys Gln
   545                 550                 555                 560
   Ala His Gly Leu Thr Pro Ala Gln Val Val Ala Ile Ala Ser His Asp
                   565                 570                 575
   Gly Gly Lys Gln Ala Leu Glu Thr Val Gln Arg Leu Leu Pro Val Leu
               580                 585                 590
   Cys Gln Asp His Gly Leu Thr Pro Asp Gln Val Val Ala Ile Ala Ser
           595                 600                 605
   Asn Ile Gly Gly Lys Gln Ala Leu Glu Thr Val Gln Arg Leu Leu Pro
       610                 615                 620
   Val Leu Cys Gln Asp His Gly Leu Thr Pro Glu Gln Val Val Ala Ile
   625                 630                 635                 640
   Ala Ser His Asp Gly Gly Lys Gln Ala Leu Glu Thr Val Gln Arg Leu
                   645                 650                 655
   Leu Pro Val Leu Cys Gln Ala His Gly Leu Thr Pro Asp Gln Val Val
               660                 665                 670
   Ala Ile Ala Ser Asn Gly Gly Gly Lys Gln Ala Leu Glu Thr Val Gln
           675                 680                 685
   Arg Leu Leu Pro Val Leu Cys Gln Ala His Gly Leu Thr Pro Ala Gln
       690                 695                 700
   Val Val Ala Ile Ala Ser Asn Asn Gly Gly Lys Gln Ala Leu Glu Thr
   705                 710                 715                 720
   Val Gln Arg Leu Leu Pro Val Leu Cys Gln Asp His Gly Leu Thr Pro
                   725                 730                 735
   Asp Gln Val Val Ala Ile Ala Ser Asn Asn Gly Gly Lys Gln Ala Leu
               740                 745                 750
   Glu Thr Val Gln Arg Leu Leu Pro Val Leu Cys Gln Asp His Gly Leu
           755                 760                 765
   Thr Pro Glu Gln Val Val Ala Ile Ala Ser Asn Asn Gly Gly Lys Gln
       770                 775                 780
   Ala Leu Glu Thr Val Gln Arg Leu Leu Pro Val Leu Cys Gln Ala His
   785                 790                 795                 800
   Gly Leu Thr Pro Asp Gln Val Val Ala Ile Ala Ser Asn Ile Gly Gly
                   805                 810                 815
   Lys Gln Ala Leu Glu Thr Val Gln Arg Leu Leu Pro Val Leu Cys Gln
               820                 825                 830
   Ala His Gly Leu Thr Pro Glu Gln Val Val Ala Ile Ala Ser Asn Ile
           835                 840                 845
   Gly Gly Arg Pro Ala Leu Glu Ser Ile Val Ala Gln Leu Ser Arg Pro
       850                 855                 860
   Asp Pro Ala Leu Ala Ala Leu Thr Asn Asp His Leu Val Ala Leu Ala
   865                 870                 875                 880
   Cys Leu Gly Gly Arg Pro Ala Met Asp Ala Val Lys Lys Gly Leu Pro
                   885                 890                 895
   His Ala Pro Glu Leu Ile Arg Ser Gln Leu Val Lys Ser Glu Leu Glu
               900                 905                 910
   Glu Lys Lys Ser Glu Leu Arg His Lys Leu Lys Tyr Val Pro His Glu
           915                 920                 925
   Tyr Ile Glu Leu Ile Glu Ile Ala Arg Asn Ser Thr Gln Asp Arg Ile
       930                 935                 940
   Leu Glu Met Lys Val Met Glu Phe Phe Met Lys Val Tyr Gly Tyr Arg
   945                 950                 955                 960
   Gly Lys His Leu Gly Gly Ser Arg Lys Pro Asp Gly Ala Ile Tyr Thr
                   965                 970                 975
   Val Gly Ser Pro Ile Asp Tyr Gly Val Ile Val Asp Thr Lys Ala Tyr
               980                 985                 990
   Ser Gly Gly Tyr Asn Leu Pro Ile  Gly Gln Ala Asp Glu  Met Gln Arg
           995                 1000                1005
   Tyr Val  Glu Glu Asn Gln Thr  Arg Asn Lys His Ile  Asn Pro Asn
       1010                1015                1020
   Glu Trp  Trp Lys Val Tyr Pro  Ser Ser Val Thr Glu  Phe Lys Phe
       1025                1030                1035
   Leu Phe  Val Ser Gly His Phe  Lys Gly Asn Tyr Lys  Ala Gln Leu
       1040                1045                1050
   Thr Arg  Leu Asn His Ile Thr  Asn Cys Asn Gly Ala  Val Leu Ser
       1055                1060                1065
   Val Glu  Glu Leu Leu Ile Gly  Gly Glu Met Ile Lys  Ala Gly Thr
       1070                1075                1080
   Leu Thr  Leu Glu Glu Val Arg  Arg Lys Phe Asn Asn  Gly Glu Ile
       1085                1090                1095
   Asn Phe
       1100
<210> 3
   <211> 8159
   <212> DNA
   <213> Artificial Sequence
<220>
   <223> Right TALEN
<220>
   <221> CDS
   <222> (5131)..(8127)
<400> 3 aaaatcagcc tcgactgtgc cttctagttg cc 8159
<210> 4
   <211> 998
   <212> PRT
   <213> Artificial Sequence
<220>
   <223> Synthetic Construct
<400> 4
   Met Ala Ser Ser Pro Pro Lys Lys Lys Arg Lys Val Ala Ala Ala Asp
   1               5                   10                  15
   Tyr Lys Asp Asp Asp Asp Lys Ser Trp Lys Asp Ala Ser Gly Trp Ser
               20                  25                  30
   Arg Met His Ala Ala Pro Arg Arg Arg Ala Ala Gln Pro Ser Asp Ala
           35                  40                  45
   Ser Pro Ala Ala Gln Val Asp Leu Arg Thr Leu Gly Tyr Ser Gln Gln
       50                  55                  60
   Gln Gln Glu Lys Ile Lys Pro Lys Val Arg Ser Thr Val Ala Gln His
   65                  70                  75                  80
   His Glu Ala Leu Val Gly His Gly Phe Thr His Ala His Ile Val Ala
                   85                  90                  95
   Leu Ser Gln His Pro Ala Ala Leu Gly Thr Val Ala Val Thr Tyr Gln
               100                 105                 110
   His Ile Ile Thr Ala Leu Pro Glu Ala Thr His Glu Asp Ile Val Gly
           115                 120                 125
   Val Gly Lys Gln Trp Ser Gly Ala Arg Ala Leu Glu Ala Leu Leu Thr
       130                 135                 140
   Asp Ala Gly Glu Leu Arg Gly Pro Pro Leu Gln Leu Asp Thr Gly Gln
   145                 150                 155                 160
   Leu Val Lys Ile Ala Lys Arg Gly Gly Val Thr Ala Met Glu Ala Val
                   165                 170                 175
   His Ala Ser Arg Asn Ala Leu Thr Gly Ala Pro Leu Asn Leu Thr Pro
               180                 185                 190
   Asp Gln Val Val Ala Ile Ala Ser His Asp Gly Gly Lys Gln Ala Leu
           195                 200                 205
   Glu Thr Val Gln Arg Leu Leu Pro Val Leu Cys Gln Asp His Gly Leu
       210                 215                 220
   Thr Pro Glu Gln Val Val Ala Ile Ala Ser Asn Gly Gly Gly Lys Gln
   225                 230                 235                 240
   Ala Leu Glu Thr Val Gln Arg Leu Leu Pro Val Leu Cys Gln Ala His
                   245                 250                 255
   Gly Leu Thr Pro Asp Gln Val Val Ala Ile Ala Ser His Asp Gly Gly
               260                 265                 270
   Lys Gln Ala Leu Glu Thr Val Gln Arg Leu Leu Pro Val Leu Cys Gln
           275                 280                 285
   Ala His Gly Leu Thr Pro Ala Gln Val Val Ala Ile Ala Ser Asn Ile
       290                 295                 300
   Gly Gly Lys Gln Ala Leu Glu Thr Val Gln Arg Leu Leu Pro Val Leu
   305                 310                 315                 320
   Cys Gln Asp His Gly Leu Thr Pro Asp Gln Val Val Ala Ile Ala Ser
                   325                 330                 335
   Asn Gly Gly Gly Lys Gln Ala Leu Glu Thr Val Gln Arg Leu Leu Pro
               340                 345                 350
   Val Leu Cys Gln Asp His Gly Leu Thr Pro Glu Gln Val Val Ala Ile
           355                 360                 365
   Ala Ser His Asp Gly Gly Lys Gln Ala Leu Glu Thr Val Gln Arg Leu
       370                 375                 380
   Leu Pro Val Leu Cys Gln Ala His Gly Leu Thr Pro Asp Gln Val Val
   385                 390                 395                 400
   Ala Ile Ala Ser Asn Gly Gly Gly Lys Gln Ala Leu Glu Thr Val Gln
                   405                 410                 415
   Arg Leu Leu Pro Val Leu Cys Gln Ala His Gly Leu Thr Pro Ala Gln
               420                 425                 430
   Val Val Ala Ile Ala Ser His Asp Gly Gly Lys Gln Ala Leu Glu Thr
           435                 440                 445
   Val Gln Arg Leu Leu Pro Val Leu Cys Gln Asp His Gly Leu Thr Pro
       450                 455                 460
   Asp Gln Val Val Ala Ile Ala Ser His Asp Gly Gly Lys Gln Ala Leu
   465                 470                 475                 480
   Glu Thr Val Gln Arg Leu Leu Pro Val Leu Cys Gln Asp His Gly Leu
                   485                 490                 495
   Thr Pro Glu Gln Val Val Ala Ile Ala Ser His Asp Gly Gly Lys Gln
               500                 505                 510
   Ala Leu Glu Thr Val Gln Arg Leu Leu Pro Val Leu Cys Gln Ala His
           515                 520                 525
   Gly Leu Thr Pro Asp Gln Val Val Ala Ile Ala Ser Asn Asn Gly Gly
       530                 535                 540
   Lys Gln Ala Leu Glu Thr Val Gln Arg Leu Leu Pro Val Leu Cys Gln
   545                 550                 555                 560
   Ala His Gly Leu Thr Pro Ala Gln Val Val Ala Ile Ala Ser Asn Gly
                   565                 570                 575
   Gly Gly Lys Gln Ala Leu Glu Thr Val Gln Arg Leu Leu Pro Val Leu
               580                 585                 590
   Cys Gln Asp His Gly Leu Thr Pro Asp Gln Val Val Ala Ile Ala Ser
           595                 600                 605
   Asn Asn Gly Gly Lys Gln Ala Leu Glu Thr Val Gln Arg Leu Leu Pro
       610                 615                 620
   Val Leu Cys Gln Asp His Gly Leu Thr Pro Glu Gln Val Val Ala Ile
   625                 630                 635                 640
   Ala Ser Asn Ile Gly Gly Lys Gln Ala Leu Glu Thr Val Gln Arg Leu
                   645                 650                 655
   Leu Pro Val Leu Cys Gln Ala His Gly Leu Thr Pro Asp Gln Val Val
               660                 665                 670
   Ala Ile Ala Ser Asn Asn Gly Gly Lys Gln Ala Leu Glu Thr Val Gln
           675                 680                 685
   Arg Leu Leu Pro Val Leu Cys Gln Ala His Gly Leu Thr Pro Ala Gln
       690                 695                 700
   Val Val Ala Ile Ala Ser His Asp Gly Gly Lys Gln Ala Leu Glu Thr
   705                 710                 715                 720
   Val Gln Arg Leu Leu Pro Val Leu Cys Gln Asp His Gly Leu Thr Pro
                   725                 730                 735
   Glu Gln Val Val Ala Ile Ala Ser Asn Gly Gly Gly Arg Pro Ala Leu
               740                 745                 750
   Glu Ser Ile Val Ala Gln Leu Ser Arg Pro Asp Pro Ala Leu Ala Ala
           755                 760                 765
   Leu Thr Asn Asp His Leu Val Ala Leu Ala Cys Leu Gly Gly Arg Pro
       770                 775                 780
   Ala Met Asp Ala Val Lys Lys Gly Leu Pro His Ala Pro Glu Leu Ile
   785                 790                 795                 800
   Arg Ser Gln Leu Val Lys Ser Glu Leu Glu Glu Lys Lys Ser Glu Leu
                   805                 810                 815
   Arg His Lys Leu Lys Tyr Val Pro His Glu Tyr Ile Glu Leu Ile Glu
               820                 825                 830
   Ile Ala Arg Asn Ser Thr Gln Asp Arg Ile Leu Glu Met Lys Val Met
           835                 840                 845
   Glu Phe Phe Met Lys Val Tyr Gly Tyr Arg Gly Lys His Leu Gly Gly
       850                 855                 860
   Ser Arg Lys Pro Asp Gly Ala Ile Tyr Thr Val Gly Ser Pro Ile Asp
   865                 870                 875                 880
   Tyr Gly Val Ile Val Asp Thr Lys Ala Tyr Ser Gly Gly Tyr Asn Leu
                   885                 890                 895
   Pro Ile Gly Gln Ala Asp Glu Met Gln Arg Tyr Val Glu Glu Asn Gln
               900                 905                 910
   Thr Arg Asn Lys His Ile Asn Pro Asn Glu Trp Trp Lys Val Tyr Pro
           915                 920                 925
   Ser Ser Val Thr Glu Phe Lys Phe Leu Phe Val Ser Gly His Phe Lys
       930                 935                 940
   Gly Asn Tyr Lys Ala Gln Leu Thr Arg Leu Asn His Ile Thr Asn Cys
   945                 950                 955                 960
   Asn Gly Ala Val Leu Ser Val Glu Glu Leu Leu Ile Gly Gly Glu Met
                   965                 970                 975
   Ile Lys Ala Gly Thr Leu Thr Leu Glu Glu Val Arg Arg Lys Phe Asn
               980                 985                 990
   Asn Gly Glu Ile Asn Phe
           995
<210> 5
   <211> 4860
   <212> DNA
   <213> Artificial Sequence
<220>
   <223> donor vector
<220>
   <221> CDS
   <222> (98)..(817)
<400> 5
   cgccattctg cctggggacg tcggagcaag cttgatttag gtgacactat agaatacaag        60
   ctacttgttc tttttgcagg atcccatcga tgccacc atg gtg agc aag ggc gag        115
                                            Met Val Ser Lys Gly Glu
                                            1               5
   gag ctg ttc acc ggg gtg gtg ccc atc ctg gtc gag ctg gac ggc gac         163
   Glu Leu Phe Thr Gly Val Val Pro Ile Leu Val Glu Leu Asp Gly Asp
               10                  15                  20
   gta aac ggc cac aag ttc agc gtg tcc ggc gag ggc gag ggc gat gcc         211
   Val Asn Gly His Lys Phe Ser Val Ser Gly Glu Gly Glu Gly Asp Ala
           25                  30                  35
   acc tac ggc aag ctg acc ctg aag ttc atc tgc acc acc ggc aag ctg         259
   Thr Tyr Gly Lys Leu Thr Leu Lys Phe Ile Cys Thr Thr Gly Lys Leu
       40                  45                  50
   ccc gtg ccc tgg ccc acc ctc gtg acc acc ctg acc tac ggc gtg cag        307
   Pro Val Pro Trp Pro Thr Leu Val Thr Thr Leu Thr Tyr Gly Val Gln
   55                  60                  65                  70
   tgc ttc agc cgc tac ccc gac cac atg aag cag cac gac ttc ttc aag        355
   Cys Phe Ser Arg Tyr Pro Asp His Met Lys Gln His Asp Phe Phe Lys
                   75                  80                  85
   tcc gcc atg ccc gaa ggc tac gtc cag gag cgc acc atc ttc ttc aag        403
   Ser Ala Met Pro Glu Gly Tyr Val Gln Glu Arg Thr Ile Phe Phe Lys
               90                  95                  100
   gac gac ggc aac tac aag acc cgc gcc gag gtg aag ttc gag ggc gac        451
   Asp Asp Gly Asn Tyr Lys Thr Arg Ala Glu Val Lys Phe Glu Gly Asp
           105                 110                 115
   acc ctg gtg aac cgc atc gag ctg aag ggc atc gac ttc aag gag gac        499
   Thr Leu Val Asn Arg Ile Glu Leu Lys Gly Ile Asp Phe Lys Glu Asp
       120                 125                 130
   ggc aac atc ctg ggg cac aag ctg gag tac aac tac aac agc cac aac        547
   Gly Asn Ile Leu Gly His Lys Leu Glu Tyr Asn Tyr Asn Ser His Asn
   135                 140                 145                 150
   gtc tat atc atg gcc gac aag cag aag aac ggc atc aag gtg aac ttc        595
   Val Tyr Ile Met Ala Asp Lys Gln Lys Asn Gly Ile Lys Val Asn Phe
                   155                 160                 165
   aag atc cgc cac aac atc gag gac ggc agc gtg cag ctc gcc gac cac        643
   Lys Ile Arg His Asn Ile Glu Asp Gly Ser Val Gln Leu Ala Asp His
               170                 175                 180
   tac cag cag aac acc ccc atc ggc gac ggc ccc gtg ctg ctg ccc gac        691
   Tyr Gln Gln Asn Thr Pro Ile Gly Asp Gly Pro Val Leu Leu Pro Asp
           185                 190                 195
   aac cac tac ctg agc acc cag tcc gcc ctg agc aaa gac ccc aac gag        739
   Asn His Tyr Leu Ser Thr Gln Ser Ala Leu Ser Lys Asp Pro Asn Glu
       200                 205                 210
   aag cgc gat cac atg gtc ctg ctg gag ttc gtg acc gcc gcc ggg atc        787
   Lys Arg Asp His Met Val Leu Leu Glu Phe Val Thr Ala Ala Gly Ile
   215                 220                 225                 230
   act ctc ggc atg gac gag ctg tac aag taa tctagaacta tagtgagtcg          837
   Thr Leu Gly Met Asp Glu Leu Tyr Lys
                   235
   tattacgtag atccagacat gataagatac attgatgagt ttggacaaac cacaactaga      897
   atgcagtgaa aaaaatgctt tatttgtgaa atttgtgatg ctattgcttt atttgtaacc      957
   attataagct gcaataaaca agttaacaac aacaattgca ttcattttat gtttcaggtt     1017
   cagggggagg tgtgggaggt tttttaattc gcggcgcgcc gcggcgccaa tgcattgggc    1077
   ccggtaccca gcttttgttc cctttagtga gggttaatac ttcttgctgc actgggaatt     1137
   cagaaaacat gagagctcac gggagatgag tgcgcgcttg gcgtaatcat ggtcatagct     1197
   gtttcctgtg tgaaattgtt atccgctcac aattccacac aacatacgag ccggaagcat     1257
   aaagtgtaaa gcctggggtg cctaatgagt gagctaactc acattaattg cgttgcgctc     1317
   actgcccgct ttccagtcgg gaaacctgtc gtgccagctg cattaatgaa tcggccaacg     1377
   cgcggggaga ggcggtttgc gtattgggcg ctcttccgct tcctcgctca ctgactcgct     1437
   gcgctcggtc gttcggctgc ggcgagcggt atcagctcac tcaaaggcgg taatacggtt     1497
   atccacagaa tcaggggata acgcaggaaa gaacatgtga gcaaaaggcc agcaaaaggc     1557
   caggaaccgt aaaaaggccg cgttgctggc gtttttccat aggctccgcc cccctgacga     1617
   gcatcacaaa aatcgacgct caagtcagag gtggcgaaac ccgacaggac tataaagata    1677
   ccaggcgttt ccccctggaa gctccctcgt gcgctctcct gttccgaccc tgccgcttac    1737
   cggatacctg tccgcctttc tcccttcggg aagcgtggcg ctttctcata gctcacgctg    1797
   taggtatctc agttcggtgt aggtcgttcg ctccaagctg ggctgtgtgc acgaaccccc    1857
   cgttcagccc gaccgctgcg ccttatccgg taactatcgt cttgagtcca acccggtaag    1917
   acacgactta tcgccactgg cagcagccac tggtaacagg attagcagag cgaggtatgt    1977
   aggcggtgct acagagttct tgaagtggtg gcctaactac ggctacacta gaaggacagt    2037
   atttggtatc tgcgctctgc tgaagccagt taccttcgga aaaagagttg gtagctcttg    2097
   atccggcaaa caaaccaccg ctggtagcgg tggttttttt gtttgcaagc agcagattac    2157
   gcgcagaaaa aaaggatctc aagaagatcc tttgatcttt tctacggggt ctgacgctca    2217
   gtggaacgaa aactcacgtt aagggatttt ggtcatgaga ttatcaaaaa ggatcttcac    2277
   ctagatcctt ttaaattaaa aatgaagttt taaatcaatc taaagtatat atgagtaaac    2337
   ttggtctgac agttaccaat gcttaatcag tgaggcacct atctcagcga tctgtctatt    2397
   tcgttcatcc atagttgcct gactccccgt cgtgtagata actacgatac gggagggctt    2457
   accatctggc cccagtgctg caatgatacc gcgagaccca cgctcaccgg ctccagattt    2517
   atcagcaata aaccagccag ccggaagggc cgagcgcaga agtggtcctg caactttatc    2577
   cgcctccatc cagtctatta attgttgccg ggaagctaga gtaagtagtt cgccagttaa    2637
   tagtttgcgc aacgttgttg ccattgctac aggcatcgtg gtgtcacgct cgtcgtttgg    2697
   tatggcttca ttcagctccg gttcccaacg atcaaggcga gttacatgat cccccatgtt    2757
   gtgcaaaaaa gcggttagct ccttcggtcc tccgatcgtt gtcagaagta agttggccgc    2817
   agtgttatca ctcatggtta tggcagcact gcataattct cttactgtca tgccatccgt    2877
   aagatgcttt tctgtgactg gtgagtactc aaccaagtca ttctgagaat agtgtatgcg    2937
   gcgaccgagt tgctcttgcc cggcgtcaat acgggataat accgcgccac atagcagaac    2997
   tttaaaagtg ctcatcattg gaaaacgttc ttcggggcga aaactctcaa ggatcttacc    3057
   gctgttgaga tccagttcga tgtaacccac tcgtgcaccc aactgatctt cagcatcttt    3117
   tactttcacc agcgtttctg ggtgagcaaa aacaggaagg caaaatgccg caaaaaaggg    3177
   aataagggcg acacggaaat gttgaatact catactcttc ctttttcaat attattgaag    3237
   catttatcag ggttattgtc tcatgagcgg atacatattt gaatgtattt agaaaaataa    3297
   acaaataggg gttccgcgca catttccccg aaaagtgcca cctaaattgt aagcgttaat    3357
   attttgttaa aattcgcgtt aaatttttgt taaatcagct cattttttaa ccaataggcc    3417
   gaaatcggca aaatccctta taaatcaaaa gaatagaccg agatagggtt gagtgttgtt    3477
   ccagtttgga acaagagtcc actattaaag aacgtggact ccaacgtcaa agggcgaaaa    3537
   accgtctatc agggcgatgg cccactacgt gaaccatcac cctaatcaag ttttttgggg    3597
   tcgaggtgcc gtaaagcact aaatcggaac cctaaaggga gcccccgatt tagagcttga    3657
   cggggaaagc cggcgaacgt ggcgagaaag gaagggaaga aagcgaaagg agcgggcgct    3717
   agggcgctgg caagtgtagc ggtcacgctg cgcgtaacca ccacacccgc cgcgcttaat    3777
   gcgccgctac agggcgcgtc ccattcgcca ttcaggctgc gcaactgttg ggaagggcga    3837
   tcggtgcggg cctcttcgct attacgccag tcgatcgacc atagccaatt caatatggcg    3897
   tatatggact catgccaatt caatatggtg gatctggacc tgtgccaatt caatatggcg    3957
   tatatggact cgtgccaatt caatatggtg gatctggacc ccagccaatt caatatggcg    4017
   gacttggcac catgccaatt caatatggcg gacttggcac tgtgccaact ggggaggggt    4077
   ctacttggca cggtgccaag tttgaggagg ggtcttggcc ctgtgccaag tccgccatat    4137
   tgaattggca tggtgccaat aatggcggcc atattggcta tatgccagga tcaatatata    4197
   ggcaatatcc aatatggccc tatgccaata tggctattgg ccaggttcaa tactatgtat    4257
   tggccctatg ccatatagta ttccatatat gggttttcct attgacgtag atagcccctc    4317
   ccaatgggcg gtcccatata ccatatatgg ggcttcctaa taccgcccat agccactccc    4377
   ccattgacgt caatggtctc tatatatggt ctttcctatt gacgtcatat gggcggtcct    4437
   attgacgtat atggcgcctc ccccattgac gtcaattacg gtaaatggcc cgcctggctc    4497
   aatgcccatt gacgtcaata ggaccaccca ccattgacgt caatgggatg gctcattgcc    4557
   cattcatatc cgttctcacg ccccctattg acgtcaatga cggtaaatgg cccacttggc      4617
   agtacatcaa tatctattaa tagtaacttg gcaagtacat tactattggc aagtacgcca    4677
   agggtacatt ggcagtactc ccattgacgt caatggcggt aaatggcccg cgatggctgc    4737
   caagtacatc cccattgacg tcaatgggga ggggcaatga cgcaaatggg cgttccattg    4797
   acgtaaatgg gcggtaggcg tgcctaatgg gaggtctata taagcaatgc tcgtttaggg    4857
   aac                                                                 4860
<210> 6
   <211> 239
   <212> PRT
   <213> Artificial Sequence
<220>
   <223> Synthetic Construct
<400> 6
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 7
   <211> 45
   <212> DNA
   <213> Artificial Sequence
<220>
   <223> Xltyr-CMVEGFP-F
<400> 7
   aacatgagag ctcacgggag atgagtgcgc gcttggcgta atcat 45
<210> 8
   <211> 47
   <212> DNA
   <213> Artificial Sequence
<220>
   <223> Xltyr-CMVEGFP-R
<400> 8
   ttctgaattc ccagtgcagc aagaagtatt aaccctcact aaaggga 47
<210> 9
   <211> 25
   <212> DNA
   <213> Artificial Sequence
<220>
   <223> tyr-genomic F
<400> 9
   ggagaggatg gcctctggag agata 25
<210> 10
   <211> 24
   <212> DNA
   <213> Artificial Sequence
<220>
   <223> tyr-genomic R
<400> 10
   ggtgggatgg attcctccca gaag 24
<210> 11
   <211> 25
   <212> DNA
   <213> Artificial Sequence
<220>
   <223> pCS2-F
<400> 11
   ataagataca ttgatgagtt tggac 25
<210> 12
   <211> 25
   <212> DNA
   <213> Artificial Sequence
<220>
   <223> pCS2-R
<400> 12
   atgcagctgg cacgacaggt ttccc 25
<210> 13
   <211> 25
   <212> DNA
   <213> Artificial Sequence
<220>
   <223> oligonucleotide 13
<400> 13
   caccgctctc acaggccacc cccca 25
<210> 14
   <211> 25
   <212> DNA
   <213> Artificial Sequence
<220>
   <223> oligonucleotide 14
<400> 14
   aaactggggg gtggcctgtg agagc 25
<210> 15
   <211> 25
   <212> DNA
   <213> Artificial Sequence
<220>
   <223> oligonucleotide 15
<400> 15
   caccgtggat ccgtggggtg gcccc 25
<210> 16
   <211> 25
   <212> DNA
   <213> Artificial Sequence
<220>
   <223> oligonucleotide 16
<400> 16
   aaacggggcc accccacgga tccac 25
<210> 17
   <211> 24
   <212> DNA
   <213> Artificial Sequence
<220>
   <223> oligonucleotide 17
<400> 17
   caccggtgcc tgaccaaggt gccc 24
<210> 18
   <211> 24
   <212> DNA
   <213> Artificial Sequence
<220>
   <223> oligonucleotide 18
<400> 18
   aaacgggcac cttggtcagg cacc 24
<210> 19
   <211> 28
   <212> DNA
   <213> Artificial Sequence
<220>
   <223> primer 19
<400> 19
   acaccaagac agacatctct gtcccttg 28
<210> 20
   <211> 22
   <212> DNA
   <213> Artificial Sequence
<220>
   <223> primer 20
<400> 20
   atccgtatcc aatgtgggga ac 22
<210> 21
   <211> 22
   <212> DNA
   <213> Artificial Sequence
<220>
   <223> primer 21
<400> 21
   ccgcaacctc cccttctacg ag 22
<210> 22
   <211> 23
   <212> DNA
   <213> Artificial Sequence
<220>
   <223> primer 22
<400> 22
   tcagcaggtc aaggggagga atg 23
<210> 23
   <211> 5966
   <212> DNA
   <213> Artificial Sequence
<220>
   <223> donor vector
<400> 23
   ctcatgacca aaatccctta acgtgagtta cgcgcgcgtc gttccactga gcgtcagacc       60
   ccgtagaaaa gatcaaagga tcttcttgag atcctttttt tctgcgcgta atctgctgct      120
   tgcaaacaaa aaaaccaccg ctaccagcgg tggtttgttt gccggatcaa gagctaccaa      180
   ctctttttcc gaaggtaact ggcttcagca gagcgcagat accaaatact gttcttctag      240
   tgtagccgta gttagcccac cacttcaaga actctgtagc accgcctaca tacctcgctc      300
   tgctaatcct gttaccagtg gctgctgcca gtggcgataa gtcgtgtctt accgggttgg      360
   actcaagacg atagttaccg gataaggcgc agcggtcggg ctgaacgggg ggttcgtgca      420
   cacagcccag cttggagcga acgacctaca ccgaactgag atacctacag cgtgagctat      480
   gagaaagcgc cacgcttccc gaagggagaa aggcggacag gtatccggta agcggcaggg      540
   tcggaacagg agagcgcacg agggagcttc cagggggaaa cgcctggtat ctttatagtc      600
   ctgtcgggtt tcgccacctc tgacttgagc gtcgattttt gtgatgctcg tcaggggggc      660
   ggagcctatg gaaaaacgcc agcaacgcgg cctttttacg gttcctggcc ttttgctggc      720
   cttttgctca catgttcttt cctgcgttat cccctgattc tgtggataac cgtattaccg      780
   cctttgagtg agctgatacc gctcgccgca gccgaacgac cgagcgcagc gagtcagtga      840
   gcgaggaagc ggaaggcgag agtagggaac tgccaggcat caaactaagc agaaggcccc      900
   tgacggatgg cctttttgcg tttctacaaa ctctttctgt gttgtaaaac gacggccagt      960
   cttaagctcg ggccccctgg gcggttctga taacgagtaa tcgttaatcc gcaaataacg     1020
   taaaaacccg cttcggcggg tttttttatg gggggagttt agggaaagag catttgtcag     1080
   aatatttaag ggcgcctgtc actttgcttg atatatgaga attatttaac cttataaatg     1140
   agaaaaaagc aacgcacttt aaataagata cgttgctttt tcgattgatg aacacctata     1200
   attaaactat tcatctatta tttatgattt tttgtatata caatatttct agtttgttaa     1260
   agagaattaa gaaaataaat ctcgaaaata ataaagggaa aatcagtttt tgatatcaaa     1320
   attatacatg tcaacgataa tacaaaatat aatacaaact ataagatgtt atcagtattt     1380
   attatcattt agaataaatt ttgtgtcgcc cttaattgtg agcggataac aattacgagc     1440
   ttcatgcaca gtggcgttga cattgattat tgactagtta ttaatagtaa tcaattacgg     1500
   ggtcattagt tcatagccca tatatggagt tccgcgttac atacccgggg ccaccccacg     1560
   gatccatggt gagtaaggga gaggaagata atatggcctc ccttcccgct acgcacgaac     1620
   tccacatctt cgggtcaatc aacggtgttg acttcgacat ggtgggccag ggcaccggca     1680
   atcccaatga cggatacgaa gaactcaatt tgaagagtac aaagggcgat ctccaattct     1740
   caccttggat tctggttccc cacattggat acggatttca tcagtacctg ccgtaccccg     1800
   atgggatgag cccatttcag gctgcaatgg tagatggtag cggttaccaa gtacaccgaa     1860
   ctatgcaatt tgaggacggt gcctcactga cagtgaacta tcggtatact tacgaaggaa     1920
   gccacatcaa gggagaggca caggtcaaag gaaccggatt tccagccgac gggccagtca     1980
   tgacaaactc cctgaccgcc gcagattggt gccgcagcaa aaagacctat ccaaatgaca     2040
   agaccattat ctcgacattc aaatggagct acaccaccgg aaacggcaaa cgctatcggt     2100
   ctaccgccag gacaacctac acatttgcaa aacctatggc cgcaaactat ctgaaaaacc     2160
   agccgatgta tgtgttccga aagacggaat taaaacactc gaaaacagaa ctaaacttta     2220
   aagagtggca gaaagccttt accgacgtaa tgggcatgga cgagctgtat aagggaagcg     2280
   gagagggcag aggaagtctg ctaacatgcg gtgacgtcga ggagaatcct ggacctatga     2340
   ccgagtacaa gcccacggtg cgcctcgcca cccgcgacga cgtcccccgg gccgtacgca     2400
   ccctcgccgc cgcgttcgcc gactaccccg ccacgcgcca caccgtcgac ccggaccgcc     2460
   acatcgagcg ggtcaccgag ctgcaagaac tcttcctcac gcgcgtcggg ctcgacatcg     2520
   gcaaggtgtg ggtcgcggac gacggcgccg cggtggcggt ctggaccacg ccggagagcg     2580
   tcgaagcggg ggcggtgttc gccgagatcg gcccgcgcat ggccgagttg agcggttccc     2640
   ggctggccgc gcagcaacag atggaaggcc tcctggcgcc gcaccggccc aaggagcccg     2700
   cgtggttcct ggccaccgtc ggcgtctcgc ccgaccacca gggcaagggt ctgggcagcg     2760
   ccgtcgtgct ccccggagtg gaggcggccg agcgcgccgg ggtgcccgcc ttcctggaga     2820
   cctccgcgcc ccgcaacctc cccttctacg agcggctcgg cttcaccgtc accgccgacg     2880
   tcgaggtgcc cgaaggaccg cgcacctggt gcatgacccg caagcccggt gcctgaccaa     2940
   ggtgcccggg tctagaatgc tgatgggcta gcaaaatcag cctcgactgt gccttctagt     3000
   tgccagccat ctgttgtttg cccctccccc gtgccttcct tgaccctgga aggtgccact     3060
   cccactgtcc tttcctaata aaatgaggaa attgcatcac aacactcaac cctatctcgg     3120
   tctattcttt tgatttataa gggattttgc cgatttcggc ctattggtta aaaaatgagc     3180
   tgatttaaca aaaatttaac gcgaattaat tctgtggaat gtgtgtcagt tagggtgtgg     3240
   aaagtcccca ggctccccag caggcagaag tatgcaaagc atgcatctca attagtcagc     3300
   aaccaggtgt ggaaagtccc caggctcccc agcaggcaga agtatgcaaa gcatgcatct     3360
   caattagtca gcaaccatag tcccgcccct aactccgccc atcccgcccc taactccgcc     3420
   cagttccgcc cattctccgc cccatggctg actaattttt tttatttatg cagaggccga     3480
   ggccgcctct gcctctgagc tattccagaa gtagtgagga ggcttttttg gaggcctagg     3540
   cttttgcaaa aagctcccgg gagcttgtat atccattttc ggatctgatc agcacgtgat     3600
   gaaaaagcct gaactcaccg cgacgtctgt cgagaagttt ctgatcgaaa agttcgacag     3660
   cgtttccgac ctgatgcagc tctcggaggg cgaagaatct cgtgctttca gcttcgatgt     3720
   aggagggcgt ggatatgtcc tgcgggtaaa tagctgcgcc gatggtttct acaaagatcg     3780
   ttatgtttat cggcactttg catcggccgc gctcccgatt ccggaagtgc ttgacattgg     3840
   ggaattcagc gagagcctga cctattgcat ctcccgccgt gcacagggtg tcacgttgca     3900
   agacctgcct gaaaccgaac tgcccgctgt tctgcagccg gtcgcggagg ccatggatgc     3960
   gatcgctgcg gccgatctta gccagacgag cgggttcggc ccattcggac cgcaaggaat     4020
   cggtcaatac actacatggc gtgatttcat atgcgcgatt gctgatcccc atgtgtatca     4080
   ctggcaaact gtgatggacg acaccgtcag tgcgtccgtc gcgcaggctc tcgatgagct     4140
   gatgctttgg gccgaggact gccccgaagt ccggcacctc gtgcacgcgg atttcggctc     4200
   caacaatgtc ctgacggaca atggccgcat aacagcggtc attgactgga gcgaggcgat     4260
   gttcggggat tcccaatacg aggtcgccaa catcttcttc tggaggccgt ggttggcttg     4320
   tatggagcag cagacgcgct acttcgagcg gaggcatccg gagcttgcag gatcgccgcg     4380
   gctccgggcg tatatgctcc gcattggtct tgaccaactc tatcagagct tggttgacgg     4440
   caatttcgat gatgcagctt gggcgcaggg tcgatgcgac gcaatcgtcc gatccggagc     4500
   cgggactgtc gggcgtacac aaatcgcccg cagaagcgcg gccgtctgga ccgatggctg     4560
   tgtagaagta ctcgccgata gtggaaaccg acgccccagc actcgtccga gggcaaagga     4620
   atagcacgtg ctacgagatt tcgattccac cgccgccttc tatgaaaggt tgggcttcgg     4680
   aatcgttttc cgggacgccg gctggatgat cctccagcgc ggggatctca tgctggagtt     4740
   cttcgcccac cccaacttgt ttattgcagc ttataatggt tacaaataaa gcaatagcat     4800
   cacaaatttc acaaataaag catttttttc actgcattct agttgtggtt tgtccaaact     4860
   catcaatgta tcttatcatg tctgtatacc gtcgacctct agctagagct tggcgtaatc     4920
   atggtcatta ccaatgctta atcagtgagg cacctatctc agcgatctgt ctatttcgtt     4980
   catccatagt tgcctgactc cccgtcgtgt agataactac gatacgggag ggcttaccat     5040
   ctggccccag cgctgcgatg ataccgcgag aaccacgctc accggctccg gatttatcag     5100
   caataaacca gccagccgga agggccgagc gcagaagtgg tcctgcaact ttatccgcct     5160
   ccatccagtc tattaattgt tgccgggaag ctagagtaag tagttcgcca gttaatagtt     5220
   tgcgcaacgt tgttgccatc gctacaggca tcgtggtgtc acgctcgtcg tttggtatgg     5280
   cttcattcag ctccggttcc caacgatcaa ggcgagttac atgatccccc atgttgtgca     5340
   aaaaagcggt tagctccttc ggtcctccga tcgttgtcag aagtaagttg gccgcagtgt     5400
   tatcactcat ggttatggca gcactgcata attctcttac tgtcatgcca tccgtaagat     5460
   gcttttctgt gactggtgag tactcaacca agtcattctg agaatagtgt atgcggcgac     5520
   cgagttgctc ttgcccggcg tcaatacggg ataataccgc gccacatagc agaactttaa     5580
   aagtgctcat cattggaaaa cgttcttcgg ggcgaaaact ctcaaggatc ttaccgctgt     5640
   tgagatccag ttcgatgtaa cccactcgtg cacccaactg atcttcagca tcttttactt     5700
   tcaccagcgt ttctgggtga gcaaaaacag gaaggcaaaa tgccgcaaaa aagggaataa     5760
   gggcgacacg gaaatgttga atactcatat tcttcctttt tcaatattat tgaagcattt     5820
   atcagggtta ttgtctcatg agcggataca tatttgaatg tatttagaaa aataaacaaa     5880
   taggggtcag tgttacaacc aattaaccaa ttctgaacat tatcgcgagc ccatttatac     5940
   ctgaatatgg ctcataacac cccttg                                          5966

## Description

### Technical Field

The present invention relates to an in vitro method for inserting a desired nucleic acid into a predetermined site in a nucleic acid contained in a cell. The present disclosure also relates to a vector for the method, a kit for the method, and a cell obtained by the method. Further, the present disclosure relates to a non-human organism comprising a cell containing a desired nucleic acid and a method for producing the non-human organism

### Background Art

TALENs (TALE Nucleases), ZFNs (Zinc Finger Nucleases), and the like are known as polypeptides including a plurality of nuclease subunits formed of DNA binding domains and DNA cleavage domains (Patent Literatures 1 to 4 and Non-Patent Literature 1). As for these artificial nucleases, a plurality of adjacent DNA cleavage domains form multimers at each binding site of the DNA binding domains, and thereby catalyze double strand break of DNAs. Each of the DNA binding domains contains repeats of a plurality of DNA binding modules. Each of the DNA binding modules recognizes a specific base pair in the DNA strand. Accordingly, a specific nucleotide sequence can be specifically cleaved by appropriately designing a DNA binding module. Other known nucleases which specifically cleave the specific nucleotide sequence are an RNA-guided nuclease such as a CRISPR/Cas system (Non-Patent Literature 2) and an RNA-guided FokI nuclease with a FokI nuclease fused to the CRISPR/Cas system (FokI-dCas9) (Non-Patent Literature 3). Various genetic modifications such as gene deletion and insertion on a genomic DNA and mutation introduction are performed using errors and recombination during repair of breaks by these nucleases (refer to Patent Literatures 5 to 6 and Non-Patent Literature 4).

As methods for inserting a desired nucleic acid into a cell using an artificial nuclease, the methods described in Non-Patent Literatures 5 to 8 are known. Non-Patent Literature 5 describes a method for inserting a foreign DNA by homologous recombination using TALENs. Non-Patent Literature 6 describes a method for inserting a foreign DNA by homologous recombination using ZFNs. However, the vector used for homologous recombination is long-stranded and cannot be easily produced. Depending on the cells and organisms, the homologous recombination efficiency is sometimes low. Therefore, these methods can be used only for limited cells and organisms. In order to obtain a modified organism that stably has a cell with a desired nucleic acid inserted therein, it is effective to obtain an adult organism by introducing a target nucleic acid into an animal embryo and differentiating the embryo. However, the homologous recombination efficiency is low in the animal embryo, and thus these methods are inefficient. A known technique for introducing a foreign DNA into animal embryos is ssODN-mediated gene modification. In this technique, it is only possible to introduce a short DNA with about several 10 bp.

The method described in Non-Patent Literature 7 or 8 is a method for inserting a nucleic acid into a cell by using an artificial nuclease without using homologous recombination. Non-Patent Literature 7 discloses a method for inserting a foreign DNA by cleaving a nucleic acid in a cell and a foreign DNA to be inserted using the ZFNs and TALENs, and joining the cleaved sites of the nucleic acid and the foreign DNA by the action of non-homologous end joining (NHEJ). However, the method described in Non-Patent Literature 7 does not control the direction of the nucleic acid to be inserted, and the junction of the nucleic acid to be inserted is not accurate. In the method described in Non-Patent Literature 8, a single-stranded end formed from the nucleic acid in the cell by nuclease cleavage is joined to a single-stranded end formed from the foreign DNA by annealing them, in order to achieve the control of direction and accurate joining. However, the method described in Non-Patent Literature 8 requires use of heterodimeric ZFNs and heterodimeric TALENs in order to prevent a DNA after insertion from being cleaved again, and a highly-active homodimeric artificial nuclease cannot be used in this method. The method described in Non-Patent Literature 8 is not used to insert the desired nucleic acid into animal embryos. Further, in the method described in Non-Patent Literature 8, the single-stranded end is frequently annealed to a wrong site, and a cell in which a nucleic acid is accurately inserted is not frequently obtained. In this regard, Non-Patent Literatures 5 to 8 do not describe a method of using an RNA-guided nuclease such as a CRISPR/Cas system or an RNA-guided FokI nuclease such as FokI-dCas9.

### Citation List

### Patent Literatures

Patent Literature 1: PCT International Publication No. WO 2011-072246
Patent Literature 2: PCT International Publication No. WO 2011-154393
Patent Literature 3: PCT International Publication No. WO 2011-159369
Patent Literature 4: PCT International Publication No. WO 2012-093833
Patent Literature 5: Japanese Patent Application National Publication (Laid-Open) No. 2013-513389
Patent Literature 6: Japanese Patent Application National Publication (Laid-Open) No. 2013-529083

### Non-Patent Literatures

Non-Patent Literature 1: Nat Rev Genet. 2010 Sep; 11 (9): 636-46.
Non-Patent Literature 2: Nat Protoc. 2013 Nov; 8 (11): 2281-308.
Non-Patent Literature 3: Nat Biotechnol. 2014 Jun; 32 (6): 569-76.
Non-Patent Literature 4: Cell. 2011 Jul 22; 146 (2): 318-31.
Non-Patent Literature 5: Nat Biotechnol. 2011 Jul 7; 29 (8): 731-4.
Non-Patent Literature 6: Nat Biotechnol. 2009 Sep; 27 (9): 851-7.
Non-Patent Literature 7: Biotechnol Bioeng. 2013 Mar; 110 (3): 871-80.
Non-Patent Literature 8: Genome Res. 2013 Mar; 23 (3): 539-46.

### Summary of Invention

### Problems to be Solved by the Invention

Therefore, an object of the present invention includes to provide an in vitro method for inserting a desired nucleic acid into a predetermined site of a nucleic acid in each cell of various non-human organisms accurately and easily without requiring any complicated step such as production of a long-stranded vector, the method also enables insertion of a relatively long-stranded nucleic acid and can be used in combination with the homodimeric nuclease including a DNA cleavage domain, the RNA-guided nuclease or the RNA-guided FokI nuclease.

### Means for Solving the Problems

The present inventors focused on a region formed of a first nucleotide sequence and a region formed of a second nucleotide sequence which sandwich a predetermined site in which a nucleic acid is to be inserted, and designed a nuclease that specifically cleaves a moiety including these regions included in a nucleic acid in a cell. Further, the present inventors designed a vector including a region formed of a first nucleotide sequence, a desired nucleic acid to be inserted and a region formed of a second nucleotide sequence in the stated order in the 5'-end to 3'-end direction. Then, the present inventors introduced the designed vector into the cell, allowed the nuclease to act on the cell, and thereby effected cleavage of the predetermined site in the nucleic acid in the cell. Further, they allowed the nuclease to act on the vector, resulting in production of a nucleic acid fragment including the region formed of the first nucleotide sequence, the desired nucleic acid and the region formed of the second nucleotide sequence in the stated order in the 5'-end to 3'-end direction. As a result, in the cell, the first nucleotide sequence in the nucleic acid in the cell and the first nucleotide sequence in the vector were joined by microhomology-mediated end joining (MMEJ), and the second nucleotide sequence in the nucleic acid in the cell and the second nucleotide sequence in the vector were joined by MMEJ. Accordingly, the desired nucleic acid was accurately inserted into the predetermined site of the nucleic acid of the cell. It was possible to perform the insertion step on relatively long-stranded nucleic acids of several kb or more. The used nuclease specifically cleaves the moiety including the region formed of the first nucleotide sequence and the region formed of the second nucleotide sequence in the nucleic acid in the cell before insertion. However, the linked nucleic acid does not include a part of the moiety because of insertion of the desired nucleic acid. Thus, the nucleic acid was not cleaved again by the nuclease present in the cell and was stably maintained, and insertion of the desired nucleic acid occurred at high frequency.

According to the method, the sequences are joined by microhomology-mediated end joining which functions in many cells. Consequently, a desired nucleic acid can be accurately inserted at high frequency into cells at the developmental stage or the like with low homologous recombination efficiency. The method can be applied to a wide range of non-human organisms and cells. Further, according to the method, a vector for introducing a nuclease and a vector for inserting a nucleic acid can be simultaneously inserted into a cell and thus the operation is simple. Furthermore, according to the method, changes in the nucleic acid moiety in the cell due to microhomology-mediated end joining prevent the inserted nucleic acid from being cleaved again. As the DNA cleavage domain included in the nuclease, a highly active homodimeric domain can also be used, and a wide range of experimental materials can be selected.

That is, according to a first aspect of the present disclosure there is provided a vector for inserting a desired nucleic acid into a predetermined site in a nucleic acid contained in a cell by a nuclease,
wherein the nucleic acid contained in the cell includes a region formed of a first nucleotide sequence, the predetermined site, and a region formed of a second nucleotide sequence in the stated order in a 5'-end to 3'-end direction,
wherein the nuclease specifically cleaves a moiety including the region formed of the first nucleotide sequence and the region formed of the second nucleotide sequence included in the cell, and
wherein the vector includes a region formed of a first nucleotide sequence, the desired nucleic acid, and a region formed of a second nucleotide sequence in the stated order in a 5'-end to 3'-end direction.

That is, according to a second aspect of the present disclosure there is provided a vector for inserting a desired nucleic acid into a predetermined site in a nucleic acid contained in a cell by a nuclease including a first DNA binding domain and a second DNA binding domain,
wherein the nucleic acid contained in the cell includes a region formed of a first nucleotide sequence, the predetermined site, and a region formed of a second nucleotide sequence in the stated order in a 5'-end to 3'-end direction,
wherein the region formed of the first nucleotide sequence, the predetermined site and the region formed of the second nucleotide sequence in the nucleic acid contained in the cell are each located between a region formed of a nucleotide sequence recognized by the first DNA binding domain and a region formed of a nucleotide sequence recognized by the second DNA binding domain,
wherein the vector includes a region formed of a first nucleotide sequence, the desired nucleic acid, and a region formed of a second nucleotide sequence in the stated order in a 5'-end to 3'-end direction,
wherein the region formed of the first nucleotide sequence and the region formed of the second nucleotide sequence in the vector are each located between a region formed of a nucleotide sequence recognized by the first DNA binding domain and a region formed of a nucleotide sequence recognized by the second DNA binding domain, and
wherein the vector produces a nucleic acid fragment including the region formed of the first nucleotide sequence, the desired nucleic acid, and the region formed of the second nucleotide sequence in the stated order in the 5'-end to 3'-end direction by the nuclease.

Further, according to a third aspect of the present disclosure there is provided the vector according to the first or second aspect, wherein the first nucleotide sequence in the nucleic acid contained in the cell and the first nucleotide sequence in the vector are joined by microhomology-mediated end joining (MMEJ), and the second nucleotide sequence in the nucleic acid contained in the cell and the second nucleotide sequence in the vector are joined by MMEJ, whereby the desired nucleic acid is inserted.

Further, according to a fourth aspect of the present disclosure there is provided the vector according to the second aspect, wherein the nuclease is a homodimeric nuclease and the vector is a circular vector.

Further, according to a fifth aspect of the present disclosure there is provided the vector according to the first aspect, wherein the nuclease is a Cas9 nuclease.

Further, according to a sixth aspect of the present disclosure there is provided the vector according to the second aspect, wherein the nuclease is a TALEN.

Further, according to a seventh aspect of the present disclosure there is provided a kit for inserting a desired nucleic acid into a predetermined site in a nucleic acid contained in a cell, comprising the vector according to any one of the first to sixth aspects and a vector for expressing a nuclease.

Further, according to the present invention, there is provided a method for inserting a desired nucleic acid into a predetermined site in a nucleic acid contained in a cell, including a step of introducing the vector according to any one of the first to sixth aspects and a vector for expressing a nuclease into a cell.

Further, according to a eight aspect of the present disclosure there is provided a cell obtained by the method of the present invention.

Further, according to a ninth aspect of the present disclosure there is provided a non-human organism comprising the cell according to the eight aspect.

Further, according to another aspect of the present invention, there is provided an in vitro method for producing a non-human organism comprising a desired nucleic acid, comprising a step wherein cells are produced by the method according to the invention and a step wherein the cells produced are diffrentiated.

Further, according to a tenth aspect of the present disclosure there is provided a non-human organism produced by the method according to the invention.

### Effects of the Invention

When the vector of the present disclosure is used, a desired nucleic acid can be accurately and easily inserted into a predetermined site of a nucleic acid in each cell of various non-human organisms without requiring any complicated step such as production of a long-stranded vector, without depending on homologous recombination efficiency in cells or organisms, and without causing any frame shift. Relatively long-stranded nucleic acids of several kb or more can also be inserted. The method for inserting a nucleic acid using the vector of the present disclosure can be used in combination with a nuclease including a homodimeric DNA cleavage domain with high nuclease activity. Alternatively, the method for inserting a nucleic acid using the vector of the present disclosure can be used in combination with an RNA-guided nuclease such as a CRISPR/Cas system. Further, when the vector of the present disclosure is used, it is possible to accurately design a junction and to knock-in a functional domain with in-frame. Thus, when a nucleic acid containing a gene as a label is used, the non-human organism subjected to target insertion can be easily identified by detecting expression of the gene. It is possible to easily obtain a non-human organism with a desired nucleic acid inserted therein at high frequency. Further, the method for inserting a nucleic acid using the vector of the present disclosure can be used for undifferentiated cells such as non-human embryos with low homologous recombination efficiency. Consequently, by inserting a desired nucleic acid into an undifferentiated cell using the vector of the present disclosure and differentiating the obtained undifferentiated cell, it is possible to easily obtain an adult non-human organism that stably maintains the desired nucleic acid.

### Brief Description of Drawings

[Fig. 1] Fig. 1 is a schematic view illustrating target integration to a tyr locus in the case where the whole vector containing a desired nucleic acid is inserted using TALENs.
[Fig. 2] Fig. 2 is a schematic view illustrating the case where a part of the vector containing a desired nucleic acid is inserted using TALENs.
[Fig. 3] Fig. 3 is a schematic view of the design of the vector of the present disclosure using a CRISPR/Cas system.
[Fig. 4a] Fig. 4a is a schematic view of a case where the whole vector containing a desired nucleic acid is inserted using a CRISPR/Cas system.
[Fig. 4b] Fig. 4b is a schematic view of a case where the whole vector containing a desired nucleic acid is inserted using a CRISPR/Cas system.
Fig. 5 is a schematic view of a case where the whole vector containing a desired nucleic acid is inserted using a FokI-dCas9.
[Fig. 6] Fig. 6 illustrates phenotype of each embryonic into which TALENs and a vector for target integration (TAL-PITCh vector) have been microinjected. Figs. 6 illustrates bright field images (upper row) and GFP fluorescence images (lower row) of TALEN R + vector-injected embryos (negative control group; A) and TALEN mix + vector-injected embryos (experimental group; B).
[Fig. 7] Fig. 7 illustrates percentages of phenotypes in the negative control group and the experimental group. The phenotypes are classified into four groups (Full, Half, Mosaic and Non), except for abnormal embryo (gray, Abnormal). The number of individuals is shown at the top of each graph.
[Fig. 8] Fig. 8 illustrates detection of the introduction of the donor vector (TAL-PITCh vector) into a target gene locus. The lower views are photographs of electrophoresis of PCR products using primer sets at the upstream and downstream of a target sequence of tyrTALEN, and the sides of the vector. The upper view illustrates the positions of the primers. Each of the arrows in the lower views indicate a band that shows integration of each vector. The numeric characters correspond to individual numbers of Fig. 6.

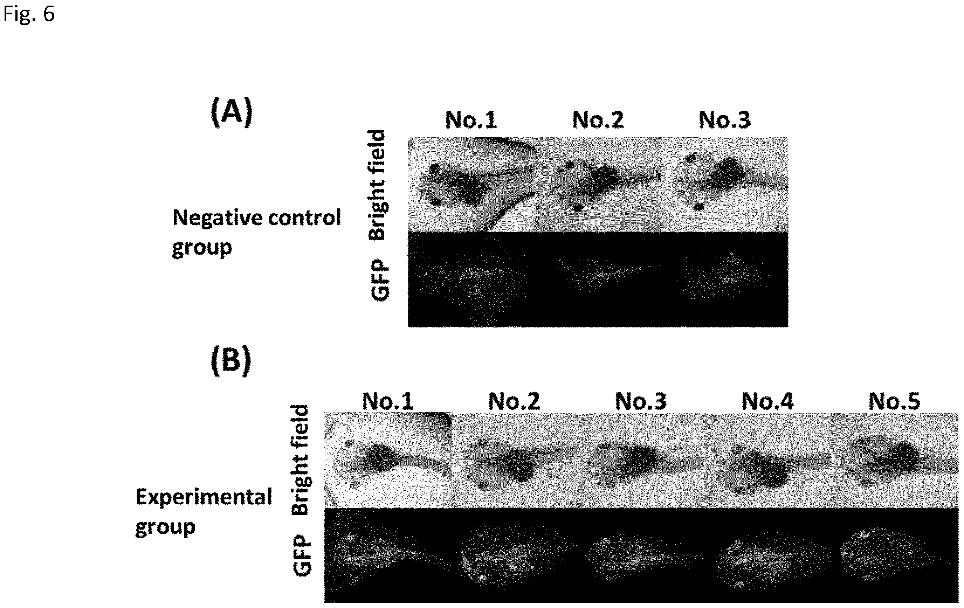

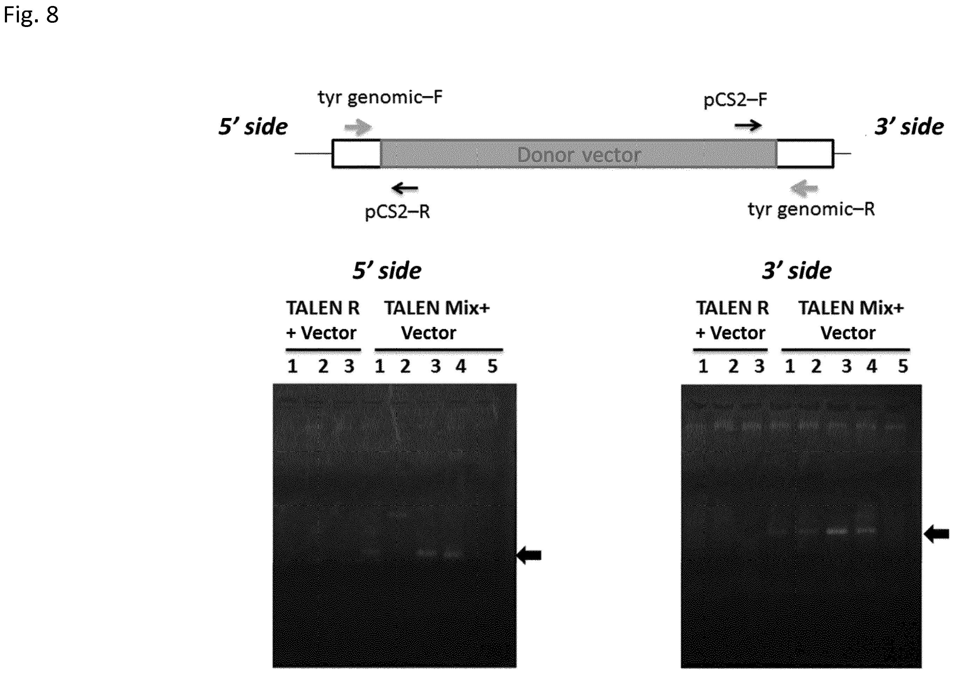

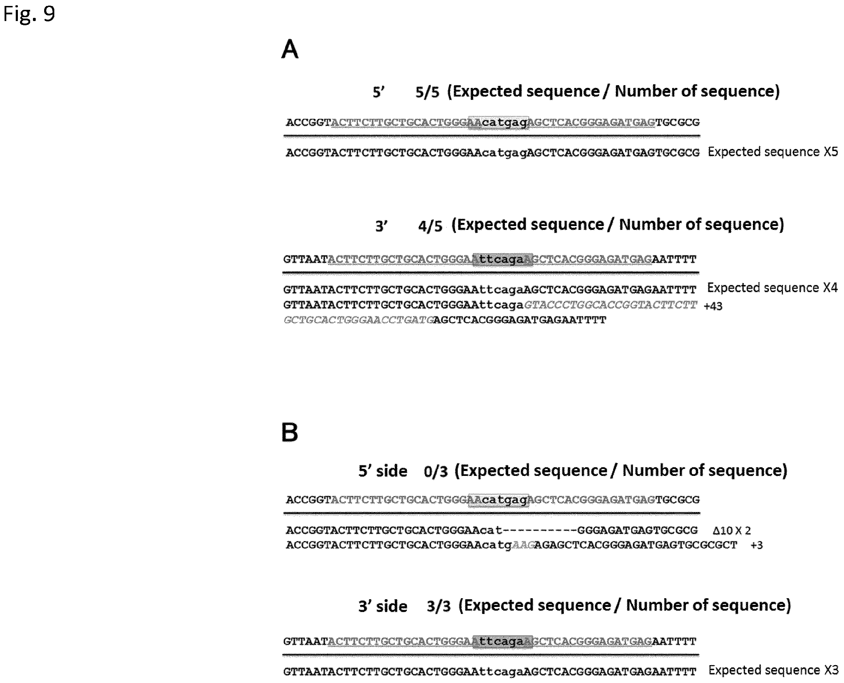

[Fig. 9] Figs. 9A and 9B illustrate sequence analysis of the junction between the insertion site and the donor vector (TAL-PITCh vector). The results of sequencing of PCR products (at the 5'-side and the 3'-side in Fig. 8) derived from Nos. 3 and 4 (Fig. 6) are shown. Sequences expected in MMEJ-dependent introduction are shown in the upper row. TALEN target sequences are underlined. Boxes near the center represent a spacer surrounding sequence shortened by MMEJ at the 5'-side and a spacer surrounding sequence shortened by MMEJ at the 3'-side, respectively. Each deletion is indicated by a dashed line (-), and each insertion is indicated by italics.
[Fig. 10] Fig. 10 is a schematic view of target integration to an FBL locus of a HEK293T cell using a CRISPR/Cas system.
[Fig. 11A] Figs. 11A and 11B illustrate the full length sequence of a donor vector (CRIS-PITCh vector). A mNeonGreen coding sequence is indicated in green, a 2A peptide coding sequence is indicated in purple and a puromycin resistance gene coding sequence is indicated in blue. A gRNA target sequence at the 5'-side and a gRNA target sequence at the 3'-side are underlined.
[Fig. 11B] Fig. 11B is a continuation of Fig. 11A.
[Fig. 12] Fig. 12 is a mNeonGreen fluorescence image showing a phenotype of a HEK293T cell in which a vector expressing three types of gRNAs and Cas9 and a donor vector (CRIS-PITCh vector) have been co-introduced.
[Fig. 13] Fig. 13 illustrates sequence analysis of the junction between the insertion site and the donor vector (CRIS-PITCh vector). The sequences expected in MMEJ-dependent introduction are shown in the upper row. Each deletion is indicated by a dashed line (-), each insertion is indicated by a double underline, and each substitution is indicated by an underline.

### Modes for Carrying Out the Invention

The vector provided by the first aspect of the present disclosure is a vector for inserting a desired nucleic acid into a predetermined site in a nucleic acid contained in a cell. Examples of the nucleic acid contained in the cell include genomic DNA in a cell. Examples of the cell origin include non-human mammals such as cow, miniature pig, pig, sheep, goat, rabbit, dog, cat, guinea pig, hamster, mouse, rat and monkey; birds; fish such as zebrafish; anphibia such as frog; reptiles; insects such as drosophila; and crustacea. Examples of the cell origin include plants such as *Arabidopsis thaliana.* The cell may be a cultured cell. The cell may be an immature cell, such as a pluripotent stem cell including an embryonic stem cell (ES cell) and an induced pluripotent stem cell (iPS cell), capable of differentiating into a more mature tissue cell. The embryonic stem cell and induced pluripotent stem cell can infinitely increase, and are useful as supply sources for a large amount of functional cells.

The cell into which the vector of the first aspect of the present disclosure is inserted includes a nucleic acid including a region formed of a first nucleotide sequence, a predetermined site in which a nucleic acid is to be inserted and a region formed of a second nucleotide sequence in the stated order in the 5'-end to 3'-end direction. The first nucleotide sequence and the second nucleotide sequence are expedient terms showing a relationship with the sequence included in the vector to be inserted. The first and second nucleotide sequences may be adjacent to the predetermined site directly or through a region consisting of a specific base sequence. When the first and second nucleotide sequences are adjacent to the predetermined site through the region consisting of a specific base sequence, the specific base sequence is preferably from 1 to 7 bases in length and more preferably from 1 to 3 bases in length. The first nucleotide sequence is preferably from 3 to 10 bases in length, more preferably from 4 to 8 bases in length, and even more preferably from 5 to 7 bases in length. The second nucleotide sequence is preferably from 3 to 10 bases in length, more preferably from 4 to 8 bases in length, and even more preferably from 5 to 7 bases in length.

The vector provided by the first aspect of the present disclosure is a vector for inserting a desired nucleic acid into a predetermined site in a nucleic acid contained in a cell using a nuclease. In the first aspect of the present disclosure the nuclease specifically cleaves the moiety including the region formed of the first nucleotide sequence and the region formed of the second nucleotide sequence in the cell. Such a nuclease is, for example, a nuclease including a first DNA binding domain and a second DNA binding domain. This nuclease will be described in the section herein in which the vector provided by the second aspect of the present disclosure is described. Examples of another nuclease which performs the specific cleavage as described above include RNA-guided nucleases such as nucleases based on the CRISPR/Cas system. In the CRISPR/Cas system, a moiety called "PAM" is essential to cleave a double strand by the Cas9 nuclease. Examples of the Cas9 nuclease include SpCas9 derived from *Streptococcus pyogenes* and StCas9 derived from *Streptococcus thermophilus.* The PAM of SpCas9 is a "5'-NGG-3'" sequence (N represents any nucleotide) and a position where the double strand is cleaved is located at a position 3 bases upstream (at the 5'-end) of the PAM. A guide RNA (gRNA) in the CRISPR/Cas system recognizes a base sequence located at the 5'-side of the position where the double strand is cleaved. Then, the position where the double strand is cleaved in the CRISPR/Cas system corresponds to the predetermined site for inserting the desired nucleic acid in the nucleic acid contained in the cell. The region formed of the first nucleotide sequence and the region formed of the second nucleotide sequence are present at both ends of the predetermined site. Accordingly, the CRISPR/Cas system using the gRNA which recognizes the base sequence located at the 5'-end of the PAM contained in a nucleic acid in a cell can specifically cleave the moiety including the region formed of the first nucleotide sequence and the region formed of the second nucleotide sequence.

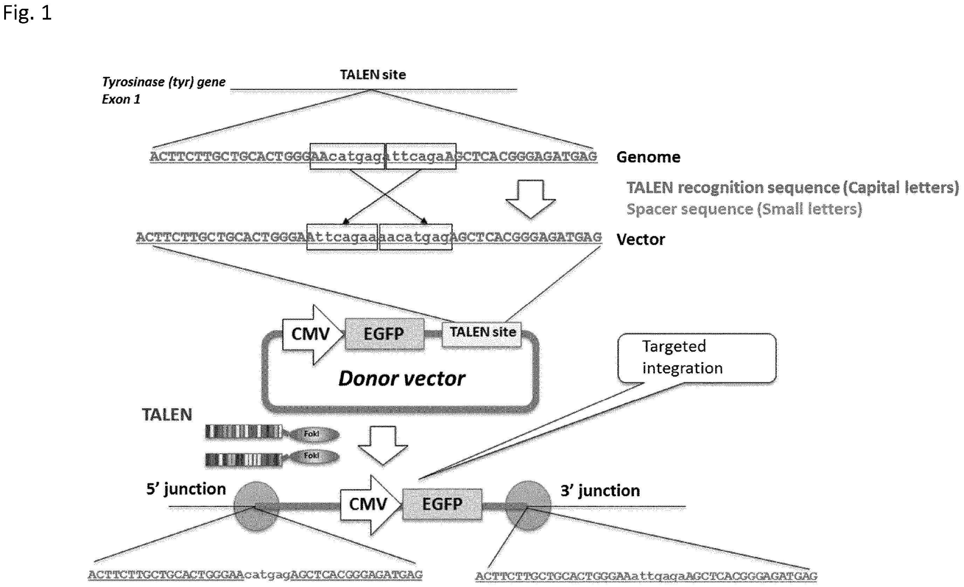

The vector provided by the first aspect of the present disclosure includes a region formed of a first nucleotide sequence, a desired nucleic acid to be inserted into a cell and a region formed of a second nucleotide sequence in the stated order in the 5'-end to 3'-end direction. The region formed of the first nucleotide sequence included in the vector is the same as the region formed of the first nucleotide sequence in the nucleic acid contained in the cell. The region formed of the second nucleotide sequence included in the vector is the same as the region formed of the second nucleotide sequence in the nucleic acid contained in the cell. A relationship between the first and second nucleotide sequences included in the vector and the first and second nucleotide sequences in the nucleic acid contained in the cell will be described using Fig. 1 as an example. "AAcatgag" contained in the TALEN site of Fig. 1 is a first nucleotide sequence. "AA" in the first nucleotide sequence is an overlap between the nucleotide sequence recognized by the first DNA binding domain and the first nucleotide sequence. "attcagaA" contained in the TALEN site of Fig. 1 is a second nucleotide sequence. The capital letter A of the second nucleotide sequence represents an overlap between the second nucleotide sequence and the nucleotide sequence recognized by the second DNA binding domain. On the other hand, "Attcagaa" contained in the donor vector of Fig. 1 is a second nucleotide sequence. The capital letter A included in the second nucleotide sequence represents an overlap between the nucleotide sequence recognized by the first DNA binding domain and the first nucleotide sequence. "aacatgaa" contained in the donor vector of Fig. 1 is a first nucleotide sequence. A sequence encoding CMV and EGFP contained in the donor vector of Fig. 1 is a desired nucleic acid to be inserted into a cell. As illustrated in the schematic view of the donor vector of Fig. 1, the donor vector will be described by defining the region formed of the first nucleotide sequence (aacatgag) as the starting point. The donor vector of Fig. 1 includes a region formed of a first nucleotide sequence, a desired nucleic acid to be inserted into a cell and a region formed of the second nucleotide sequence in the stated order in the 5'-end to 3'-end direction. The donor vector of Fig. 1 will be described in comparison to the TALEN site of Fig. 1. In the TALEN site, the 3'-end of the first nucleotide sequence is adjacent to or in contact with the 5'-end of the second nucleotide sequence. On the other hand, in the donor vector, the 3'-end of the second nucleotide sequence is adjacent to or in contact with the 5'-end of the first nucleotide sequence. In this regard, in an example of Fig. 1, a positional relationship between the first nucleotide sequence and the second nucleotide sequence in the nucleic acid in the cell is reversed, compared to a positional relationship between the first nucleotide sequence and the second nucleotide sequence in the vector. Such a relationship results from the fact that the donor vector of Fig. 1 is a circular vector and the nuclease cleaves a moiety including the region formed of the first nucleotide sequence and the region formed of the second nucleotide sequence in the vector. Thus, the vector of the first aspect of the present disclosure is preferably a circular vector. In the case where the vector of the first aspect of the present disclosure is a circular vector, the 3' -end of the second nucleotide sequence and the 5'-end of the first nucleotide sequence which are contained in the vector of the first aspect of the present disclosure are preferably adjacent or directly linked to each other. In the case where the vector of the first aspect of the present disclosure is a circular vector and the second nucleotide sequence is adjacent to the first nucleotide sequence, the 3'-end of the second nucleotide sequence is separated from the 5'-end of the first nucleotide sequence preferably by a region of from 1 to 7 bases in length, more preferably from 1 to 5 bases in length, and even more preferably from 1 to 3 bases in length.

The vector provided by the second aspect of the present disclosure is a vector for inserting a desired nucleic acid using a nuclease including a first DNA binding domain and a second DNA binding domain.

Examples of the origin of a DNA binding domain include TALEs (transcription activator-like effectors) of plant pathogen Xanthomonas and Zinc fingers. Preferably, the DNA binding domain continuously includes one or more DNA binding modules that specifically recognize base pairs from the N-terminus. One DNA binding module specifically recognizes one base pair. Therefore, the first DNA binding domain and the second DNA binding domain each recognize a region formed of a specific nucleotide sequence. The nucleotide sequence recognized by the first DNA binding domain and the nucleotide sequence recognized by the second DNA binding domain may be the same as or different from each other. The number of DNA binding modules included in the DNA binding domain is preferably from 8 to 40, more preferably from 12 to 25, and even more preferably from 15 to 20, from the viewpoint of compatibility between the level of nuclease activity and the level of DNA sequence recognition specificity of the DNA cleavage domain. The DNA binding module is, for example, a TAL effector repeat. Examples of the length of a DNA binding module include a length of from 20 to 45, a length of from 30 to 38, a length of from 32 to 36 and a length of 34. All the DNA binding modules included in the DNA binding domain are preferably identical in length. The first DNA binding domain and the second DNA binding domain are preferably identical in origin and characteristics.

In the case where the RNA-guided FokI nuclease (FokI-dCas9) is used, the FokI-dCas9 forming a complex with a gRNA corresponds to the nuclease including the DNA binding domain in the second aspect. The dCas9 is a Cas9 whose catalytic activity is inactivated. The dCas9 is guided by a gRNA recognizing a base sequence located near the site in which a double strand is cleaved, and is linked to a nucleic acid. That is, the dCas9 forming a complex with a gRNA corresponds to the DNA binding domain in the second aspect.

The nuclease including the first DNA binding domain and the second DNA binding domain preferably includes a first nuclease subunit including a first DNA binding domain and a first DNA cleavage domain and a second nuclease subunit including a second DNA binding domain and a second DNA cleavage domain.

Preferably, the first DNA cleavage domain and the second DNA cleavage domain approach each other to form a multimer after each of the first DNA binding domain and the second DNA binding domain is linked to a DNA, and acquires an improved nuclease activity. The DNA cleavage domain is, for example, a DNA cleavage domain derived from a restriction enzyme FokI. The DNA cleavage domain may be a heterodimeric DNA cleavage domain or may be a homodimeric DNA cleavage domain. When the first DNA cleavage domain and the second DNA cleavage domain approach each other, a multimer is formed and an improved nuclease activity is obtained. However, In the case where neither the multimer is formed nor the improved nuclease activity is obtained even if the first DNA cleavage domain and the first DNA cleavage domain approach each other, and neither the multimer is formed nor the improved nuclease activity is obtained even if the second DNA cleavage domain and the second DNA cleavage domain approach each other, each of the first DNA cleavage domain and the second DNA cleavage domain is a heterodimeric DNA cleavage domain. In the case where a multimer is formed and the nuclease activity is improved when the first DNA cleavage domain and the first DNA cleavage domain approach each other, the first DNA cleavage domain is a homodimeric DNA cleavage domain. In the case of using the homodimeric DNA cleavage domain, a high nuclease activity is generally obtained. The first DNA cleavage domain and the second DNA cleavage domain are preferably identical in origin and characteristics.

In the case of using a TALEN, the first DNA binding domain and the first DNA cleavage domain in the first nuclease subunit are linked by a polypeptide consisting of from 20 to 70 amino acids, from 25 to 65 amino acids or from 30 to 60 amino acids, preferably from 35 to 55 amino acids, more preferably from 40 to 50 amino acids, even more preferably from 45 to 49 amino acids, and most preferably 47 amino acids. In the case of using ZFN, the first DNA binding domain and the first DNA cleavage domain in the first nuclease subunit are linked by a polypeptide consisting of from 0 to 20 amino acids or from 2 to 10 amino acids, preferably from 3 to 9 amino acids, more preferably from 4 to 8 amino acids and even more preferably from 5 to 7 amino acids. In the case of using FokI-dCas9, the dCas9 and FokI in the first nuclease subunit are linked by a polypeptide consisting of from 1 to 20 amino acids, from 1 to 15 amino acids or from 1 to 10 amino acids, preferably from 2 to 8 amino acids, more preferably from 3 to 7 amino acids, even more preferably from 4 to 6 amino acids, and most preferably 5 amino acids. The same holds for the second nuclease subunit. The first nuclease subunit linked by such a length of polypeptide has high specificity to the length of the moiety including the region formed of the first nucleotide sequence and the region formed of the second nucleotide sequence, and specifically cleaves a spacer region having a specific length. Thus, the nucleic acid is not frequently inserted into a site outside the target site by nonspecific cleavage, and the nucleic acid joined by microhomology-mediated end joining as described later is not frequently cleaved again. This is preferable.

In the nucleic acid contained in the cell into which the vector provided by the second aspect of the present disclosure is to be inserted, the region formed of the first nucleotide sequence is located between the region formed of the nucleotide sequence recognized by the first DNA binding domain and the region formed of the nucleotide sequence recognized by the second DNA binding domain. Further, the region formed of the second nucleotide sequence is located between the region formed of the nucleotide sequence recognized by the first DNA binding domain and the region formed of the nucleotide sequence recognized by the second DNA binding domain. Further, the predetermined site is located between the region formed of the nucleotide sequence recognized by the first DNA binding domain and the region formed of the nucleotide sequence recognized by the second DNA binding domain. In the nucleic acid, a combination of two nucleotide sequences recognized by the DNA binding domain surrounding the region formed of the first nucleotide sequence may be different from a combination of two nucleotide sequences recognized by the DNA binding domain surrounding the region formed of the second nucleotide sequence. In this case, different nucleases are used as the nuclease for cleaving around the region formed of the first nucleotide sequence and the nuclease for cleaving around the region formed of the second nucleotide sequence. In the nucleic acid contained in the cell, the region formed of the nucleotide sequence recognized by the first DNA binding domain and the region formed of the nucleotide sequence recognized by the second DNA binding domain are separated by a region formed of a nucleotide sequence of preferably from 5 to 40 bases in length, more preferably from 10 to 30 bases in length, and even more preferably from 12 to 20 bases in length. The base length of the region separating both the regions may be the same as or different from the total of the base length of the first nucleotide sequence and the base length of the second nucleotide sequence. For example, in the nucleic acid contained in the cell, in the case where the following conditions are satisfied: the 3'-end of the first nucleotide sequence is directly in contact with the 5'-end of the second nucleotide sequence, there is no overlap between the nucleotide sequence recognized by the first DNA binding domain and the first nucleotide sequence, and there is no overlap between the second nucleotide sequence and the nucleotide sequence recognized by the second DNA binding domain, the base length of the region separating both the regions is the same as the total of the base length of the first nucleotide sequence and the base length of the second nucleotide sequence. However, in the case where one or more items selected from these conditions are not satisfied, the base length of the region separating both the regions is different from the total of the base length of the first nucleotide sequence and the base length of the second nucleotide sequence. The region formed of the first nucleotide sequence in the nucleic acid contained in the cell may partially overlap the region formed of the nucleotide sequence recognized by the first DNA binding domain. Further, the region formed of the second nucleotide sequence in the nucleic acid contained in the cell may partially overlap the region formed of the nucleotide sequence recognized by the second DNA binding domain. In the case where there is a partial overlap, the overlapping moiety consists of a nucleotide sequence of preferably from 1 to 6 bases in length, more preferably from 1 to 5 bases in length, and even more preferably from 2 to 4 bases in length. In the case where there is a partial overlap, the length of a moiety which separates two regions recognized by the DNA binding domain and includes the region formed of the first nucleotide sequence and the region formed of the second nucleotide sequence is greatly reduced by microhomology-mediated end joining as described later. Thus, the linked nucleic acid is hardly cleaved again and the inserted nucleic acid is more stably maintained. This is preferable.

In the vector provided by the second aspect of the present disclosure the region formed of the first nucleotide sequence and the region formed of the second nucleotide sequence are each located between the region formed of the nucleotide sequence recognized by the first DNA binding domain and the region formed of the nucleotide sequence recognized by the second DNA binding domain. In the vector, a combination of two nucleotide sequences recognized by the DNA binding domain surrounding the region formed of the first nucleotide sequence may be different from a combination of two nucleotide sequences recognized by the DNA binding domain surrounding the region formed of the second nucleotide sequence. In this case, different nucleases are used as the nuclease for cleaving around the region formed of the first nucleotide sequence and the nuclease for cleaving around the region formed of the second nucleotide sequence. In the vector, the region formed of the nucleotide sequence recognized by the first DNA binding domain may be present at the 5'-end or the 3'-end as compared to the region formed of the nucleotide sequence recognized by the second DNA binding domain. However, in the vector, the nucleotide sequence that is located at the 3'-end of the first nuclease sequence and recognized by the first DNA binding domain or the second DNA binding domain is preferably different from the sequence that is located at the 3'-end of the second nucleotide sequence in the nucleic acid contained in the cell and recognized by the first DNA binding domain or the second DNA binding domain. Further, in the vector, the nucleotide sequence that is located at the 5'-end of the second nuclease sequence and recognized by the first DNA binding domain or the second DNA binding domain is preferably different from the sequence that is located at the 5'-end of the first nucleotide sequence in the nucleic acid contained in the cell and recognized by the first DNA binding domain or the second DNA binding domain. In these cases, the frequency of cleavage occurring again after insertion of a desired nucleic acid can be further reduced by using a nuclease including a heterodimeric DNA cleavage domain in combination. In the vector, one site may be cleaved, or two or more sites may be cleaved by one or more nucleases containing a first DNA binding domain and a second DNA binding domain. The vector cleaved at two sites is, for example, a vector including a region formed of a nucleotide sequence recognized by a first DNA binding domain, a region formed of a first nucleotide sequence, a region formed of a nucleotide sequence recognized by a second DNA binding domain, a desired nucleic acid to be inserted into a cell, the region formed of the nucleotide sequence recognized by the first DNA binding domain, a region formed of a second nucleotide sequence, and the region formed of the nucleotide sequence recognized by the second DNA binding domain in the stated order in the 5'-end to 3'-end direction. In the case of using the vector cleaved at two sites, unnecessary nucleic acids contained in the vector can be removed by nuclease cleavage. Consequently, it is possible to more safely obtain a desired cell containing no unnecessary nucleic acids.

In the vector provided by the second aspect of the present disclosure the region that separates the region formed of the nucleotide sequence recognized by the first DNA binding domain from the region formed of the nucleotide sequence recognized by the second DNA binding domain and that includes the region formed of the first nucleotide sequence or the region formed of the second nucleotide sequence consists of a nucleotide sequence of preferably from 5 to 40 bases in length, more preferably from 10 to 30 bases in length, and even more preferably from 12 to 20 bases in length. In the case where the region that separates the region formed of the nucleotide sequence recognized by the first DNA binding domain from the region formed of the nucleotide sequence recognized by the second DNA binding domain includes both the first nucleotide sequence and the second nucleotide sequence, the base length of the region separating both the regions is the same or almost the same as the total of the base length of the first nucleotide sequence and the base length of the second nucleotide sequence. As described above, the first nucleotide sequence is preferably from 3 to 10 bases in length, more preferably from 4 to 8 bases in length, and even more preferably from 5 to 7 bases in length. As described above, the second nucleotide sequence is preferably from 3 to 10 bases in length, more preferably from 4 to 8 bases in length, and even more preferably from 5 to 7 bases in length. In the case where there is an overlap between the region formed of the first nucleotide sequence or the second nucleotide sequence and the region formed of the nucleotide sequence recognized by the DNA binding domain, the case where there is an overlap between the region formed of the first nucleotide sequence and the region formed of the second nucleotide sequence or the case where the region formed of the first nucleotide sequence is not directly linked to the region formed of the second nucleotide sequence, the base length of the region separating both the regions is not the same but almost the same as the total of the base length of the first nucleotide sequence and the base length of the second nucleotide sequence.

In the vector provided by the first or second aspect of the present disclosure for example, the first nucleotide sequence in the nucleic acid contained in the cell and the first nucleotide sequence in the vector are joined by microhomology-mediated end joining (MMEJ), and the second nucleotide sequence in the nucleic acid contained in the cell and the second nucleotide sequence in the vector are joined by MMEJ, whereby the desired nucleic acid is inserted into a predetermined site in the nucleic acid contained in the cell.

In the vector provided by the second aspect of the present disclosure for example, the nuclease is a homodimeric nuclease and the vector is a circular vector.

In the vector provided by the first aspect of the present disclosure for example, the nuclease is an RNA-guided nuclease such as a nuclease based on the CRISPR/Cas system. Preferably, the nuclease is a Cas9 nuclease.

In the vector provided by the second aspect of the present disclosure the nuclease is preferably a ZFN, a TALEN or FokI-dCas9, and more preferably a TALEN. The ZFN, TALEN or FokI-dCas9 may be homodimeric or heterodimeric. The nuclease is preferably a homodimeric ZFN, TALEN or FokI-dCas9, and more preferably a homodimeric TALEN.

The nucleases also include their mutants. Such a mutant may be any mutant as long as it exhibits the activity of the nuclease. The mutant is, for example, a nuclease containing the amino acid sequence in which several amino acids, such as 1, 2, 3, 4, 5, 6, 7, 8, 9, 10, 15, 20, 25, 30, 35, 40, 45 or 50 amino acids are substituted, deleted and/or added in the amino acid sequence of the nuclease.

A desired nucleic acid contained in the vector provided by the present disclosure is, for example, from 10 to 10000 bases in length and may be several kilo bases in length. The desired nucleic acid may also contain a nucleic acid encoding a gene. The gene encoded can be any gene. Examples thereof include genes encoding an enzyme converting a chemiluminescence substrate such as alkaline phosphatase, peroxidase, chloramphenicol acetyltransferase and β-galactosidase. The desired nucleic acid may contain a nucleic acid encoding a gene capable of detecting the expression level by the light signal. In this case, the presence or absence of the light signal in the cell after vector introduction is detected so that the success or failure of the insertion can be easily confirmed, and the efficiency and frequency of obtaining a cell having a desired nucleic acid inserted therein are improved. Examples of the gene capable of detecting the expression level by the light signal include genes encoding a fluorescent protein such as a green fluorescent protein (GFP), a humanized Renilla green fluorescent protein (hrGFP), an enhanced green fluorescent protein (eGFP), a yellowish green fluorescent protein (mNeonGreen), an enhanced blue fluorescent protein (eBFP), an enhanced cyan fluorescent protein (eCFP), an enhanced yellow fluorescent protein (eYFP) and a red fluorescent protein (RFP or DsRed); and genes encoding a bioluminescence protein such as firefly luciferase and Renilla luciferase.

In the vector provided by the present disclosure it is preferable that the region formed of the first nucleotide sequence is directly adjacent to the desired nucleic acid. Further, it is preferable that the desired nucleic acid is directly adjacent to the region formed of the second nucleotide sequence. In the case where the desired nucleic acid contains a functional factor such as a gene, the first and second nucleotide sequences included in the vector may encode a part of the functional factor.

The vector provided by the present disclosure may be a circular vector or a linear vector. The vector provided by the present disclosure is preferably a circular vector. Examples of the vector of the present disclosure include a plasmid vector, a cosmid vector, a viral vector and an artificial chromosome vector. Examples of the artificial chromosome vector include yeast artificial chromosome vector (YAC), bacterial artificial chromosome vector (BAC), P1 artificial chromosome vector (PAC), mouse artificial chromosome vector (MAC) and human artificial chromosome vector (HAC). Examples of the component of the vector include a nucleic acid such as a DNA and an RNA; and a nucleic acid analog such as a GNA, an LNA, a BNA, a PNA and a TNA. The vector may be modified by components other than the nucleic acid, such as saccharides.

According to the seventh aspect, the present disclosure provides a kit for inserting a desired nucleic acid into a predetermined site in a nucleic acid contained in a cell. The kit according to the seventh aspect of the present disclosure comprises the vector according to any one of the first to sixth aspects. The kit according to the seventh aspect of the present disclosure further comprises a vector for expressing a nuclease. The vector for expressing a nuclease is, for example, a vector for expressing a nuclease including a first DNA binding domain and a second DNA binding domain. Examples of the vector for expressing a nuclease include a plasmid vector, a cosmid vector, a viral vector and an artificial chromosome vector. The vector for expressing a nuclease is, for example, a vector set comprising a first vector that contains a gene encoding a first nuclease subunit including a first DNA binding domain and a first DNA cleavage domain and a second vector that contains a gene encoding a second nuclease subunit including a second DNA binding domain and a second DNA cleavage domain. Another example is a vector including both of the gene encoding the first nuclease subunit and the gene encoding the second nuclease subunit. The first and second vectors may be present in different nucleic acid fragments or identical nucleic acid fragments. In the case where different nucleases are used as the nuclease for cleaving around the region formed of the first nucleotide sequence and the nuclease for cleaving around the region formed of the second nucleotide sequence, the kit of the seventh aspect of the present disclosure comprises a plurality of the vector sets including first and second vectors. In the case of using the nuclease based on the CRISPR/Cas system as a nuclease, the kit of the seventh aspect of the present disclosure may comprise: a vector for expressing a gRNA and a nuclease for cleaving around the region formed of the first nucleotide sequence in the vector of the first aspect of the present disclosure a vector for expressing a gRNA and a nuclease for cleaving around the region formed of the second nucleotide sequence in the vector of the first aspect of the present disclosure and a a vector for expressing gRNA and a nuclease for cleaving a predetermined site in a nucleic acid contained in a cell. The vector for expressing a nuclease based on the CRISPR/Cas system may contain a vector for expressing a gRNA and a vector for expressing Cas9 per one cleavage site. The vector for expressing a gRNA and Cas9 may contain both a gene encoding a gRNA and a gene encoding Cas9. Alternatively, the vector may be a vector set including a vector containing the gene encoding a gRNA and a vector containing the gene encoding Cas9. A plurality of vectors having different functions may be present in identical nucleic acid fragments or may be present in different nucleic acid fragments.

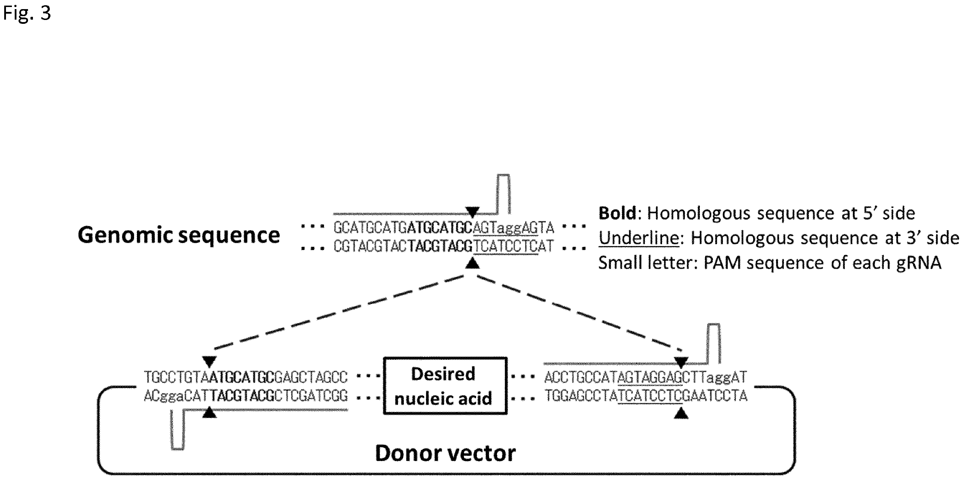

The present invention provides a method for inserting a desired nucleic acid into a predetermined site in a nucleic acid contained in a cell. The method according to the present invention comprises a step of introducing the vector according to any one of the first to sixth aspects of the present disclosure and the vector for expressing a nuclease into a cell. The vector for expressing a nuclease is, for example, a vector for expressing a nuclease including a first DNA binding domain and a second DNA binding domain as described above. Another example is a vector set including a first vector that contains a gene encoding a first nuclease subunit including a first DNA binding domain and a first DNA cleavage domain and a second vector that contains a gene encoding a second nuclease subunit including a second DNA binding domain and a second DNA cleavage domain. These vectors may be introduced into cells by allowing the vectors to be in contact with *ex vivo* cultured cells, or by administering the vectors into the living body and allowing the vectors to be indirectly in contact with cells present in the living body. These vectors can be introduced into the cells simultaneously or separately. In the case where these vectors are introduced separately into the cells, for example, a vector for expressing a nuclease may be previously introduced into a cell to produce a stable expression cell line or inducible expression cell line of the nuclease, and then, the vector according to any one of the first to sixth aspects of the present disclosure may be introduced into the produced stable expression cell line or inducible expression cell line. When the step of introduction into a cell is performed, a nuclease (such as the nuclease including the first DNA binding domain and the second DNA binding domain) functions in the cell, resulting in a nucleic acid fragment including a region formed of a first nucleotide sequence, a desired nucleic acid to be inserted into a cell and a region formed of a second nucleotide sequence in the stated order in the 5'-end to 3'-end direction from the vector. The step results in cleavage of a predetermined site in a nucleic acid in a cell. Thereafter, in the cell, the first nucleotide sequence in the nucleic acid in the cell and the first nucleotide sequence in the vector are joined by microhomology-mediated end joining (MMEJ), and the second nucleotide sequence in the nucleic acid in the cell and the second nucleotide sequence in the vector are joined by MMEJ. As a result, a desired nucleic acid is accurately inserted into a predetermined site of a nucleic acid of a cell. In the case of using the vector of the first aspect of the present disclosure the nuclease for combination use specifically cleaves the moiety including the region formed of the first nucleotide sequence and the region formed of the second nucleotide sequence in the nucleic acid in the cell before insertion. However, the linked nucleic acid does not contain the moiety because of insertion of the desired nucleic acid. For example, in the case of using the nuclease based on the CRISPR/Cas system, all the gRNA target sequences lose the PAM sequence and the sequence of 3 bases adjacent to the PAM sequence after linkage. Thus, the nucleic acid is not cleaved again by the nuclease present in the cell and is stably retained. Insertion of the desired nucleic acid occurs at high frequency. In this regard, a combination of the vector of the first aspect of the present invention and the CRISPR/Cas system such that the linked nucleic acid loses the PAM sequence or the base adjacent to the PAM sequence can be appropriately designed with reference to the first and second nucleotide sequences included in both the vector and the nucleic acid in the cell as well as the sequences adjacent to these nucleotide sequences. An example of the design is illustrated in a schematic view in Fig. 3. In the case of using the vector of the second aspect of the present disclosure the spacer region separating two DNA binding domains in the linked nucleic acid is shorter than that before linkage. Thus, the nucleic acid is not cleaved again by the nuclease present in the cell and is stably retained. Insertion of a desired nucleic acid occurs at high frequency. In this regard, the cleavage activity of the nuclease including a plurality of DNA binding domains depends on the length of the spacer region sandwiched between the regions recognized by the DNA binding domains. The nuclease specifically cleaves a spacer region having a specific length. In the linked nucleic acid, the spacer region separating two DNA binding domains consists of a nucleotide sequence of preferably from 1 to 20 bases in length, more preferably from 2 to 15 bases in length, and even more preferably from 3 to 10 bases in length.

In the present disclosure the vector for inserting a desired nucleic acid into a predetermined site in a nucleic acid contained in a cell and the vector for expressing a nuclease may be identical to or different from each other. In the case of using the nuclease based on the CRISPR/Cas system, the vector for inserting a desired nucleic acid into a predetermined site in a nucleic acid contained in a cell, the vector for expressing a nuclease and the vector for expressing a gRNA may be identical to or different from one another.

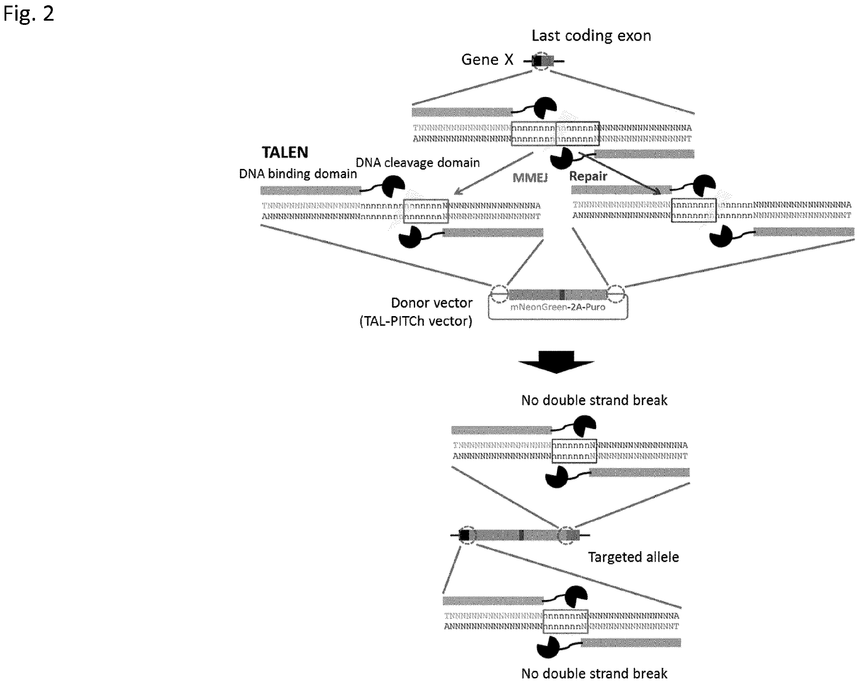

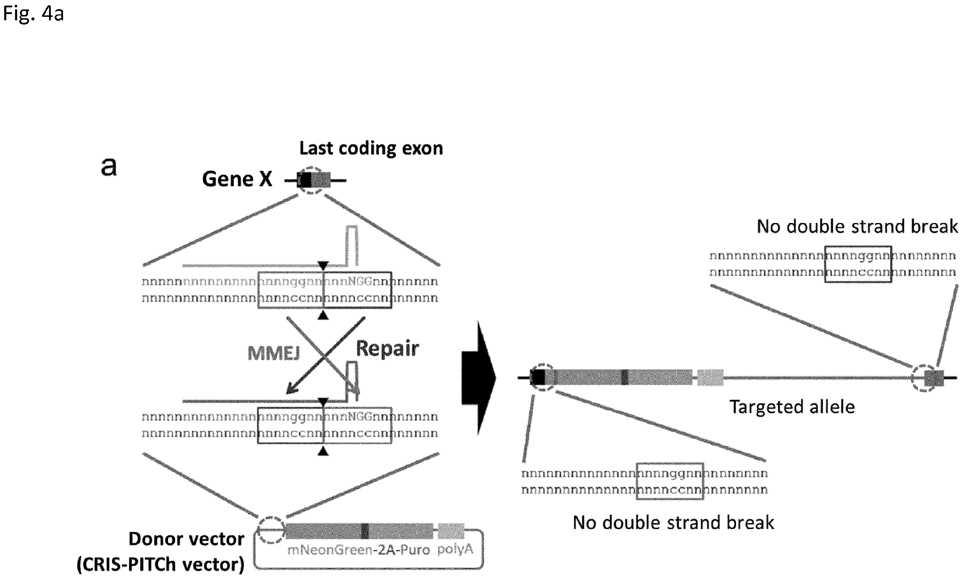

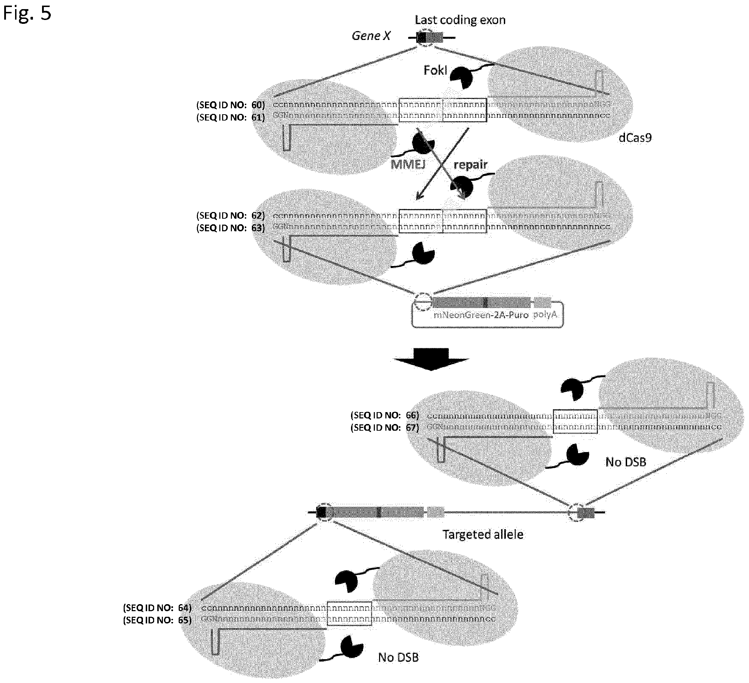

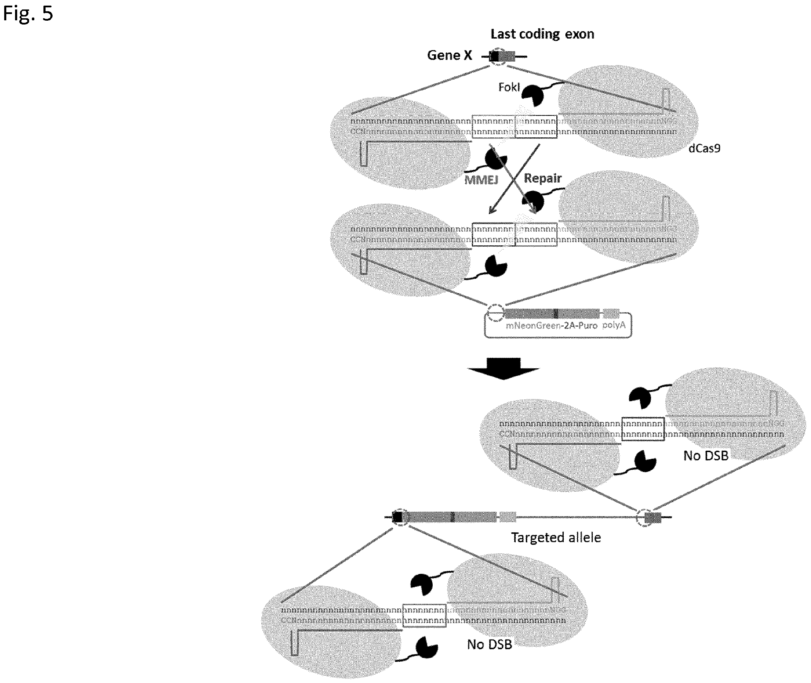

In the case where a desired nucleic acid is inserted using the vector of the present disclosure a part of the vector containing a desired nucleic acid may be inserted into a predetermined site in a nucleic acid contained in a cell. Alternatively, the whole vector containing a desired nucleic acid may be inserted into a predetermined site in a nucleic acid contained in a cell. Fig. 1 is a schematic view of a case where the whole vector containing a desired nucleic acid is inserted using TALENs. Fig. 2 is a schematic view illustrating the case where a part of the vector containing a desired nucleic acid is inserted using TALENs. Fig. 3 is a schematic view of a case where a part of the vector containing a desired nucleic acid is inserted into a predetermined site in a nucleic acid contained in a cell using the CRISPR/Cas system. Figs. 4A and 4B are each a schematic view of a case where the whole vector containing a desired nucleic acid is inserted using the CRISPR/Cas system. Fig. 5 is a schematic view of a case where the whole vector containing a desired nucleic acid is inserted using FokI-dCas9.

According to the eigth aspect, the present disclosure provides a cell obtained by the method according to the present invention. The cell of the eigth aspect of the present disclosure can be obtained by performing the introduction step in the method of the invention and then selecting the cell with the nucleic acid inserted. For example, in the case where the nucleic acid to be inserted contains a gene encoding a specific reporter protein, selection of cells can be easily performed at high frequency by detecting the expression of the reporter protein and selecting the amount of the detected expression as an indicator.

According to the ninth aspect, the present disclosure provides a non-human organism comprising the cell of the eigth aspect of the present disclosure In the method of the present invention, in the case of administering the vectors into the living body and allowing the vectors to be indirectly in contact with cells present in the living body, the non-human organism of the ninth aspect of the present disclosure is obtained.

The present invention provides a method for producing a non-human organism comprising a desired nucleic acid, comprising a step wherein cells are produced by the method of the invention and a step wherein the cells produced are differentiated. In the method of the present invention, a cell comprising a desired nucleic acid is obtained by allowing a vector to be in contact with an ex vivo cultured cell, and differentiating the obtained cell to form a non-human adult organism comprising a desired nucleic acid.

According to the tenth aspect, the present disclosure provides a non-human organism produced by the method according to the present invention. The produced non-human organism comprises a desired nucleic acid in a predetermined site of a nucleic acid contained in a cell in the non-human organism and can be used in various applications such as analysis of the functions of biological substances (e.g., genes, proteins, lipids and saccharides) depending on the function of the desired nucleic acid.

### Examples

Hereinafter, the present invention will be more specifically described with reference to examples, but the present invention is not limited thereto.

### Example 1

### Target Integration with TALEN

In this example, an expression cassette of a fluorescent protein gene was introduced (target integration) into Exon1 of a tyrosinase (tyr) gene of *Xenopus laevis* using the TALEN and the donor vector (TAL-PITCh vector).

### 1-1. Construction of TALEN:

The TALEN plasmid was constructed in the following manner. A vector constructed by In-Fusion cloning (Clontech Laboratories, Inc.) using pFUS_B6 vector (Addgene) as a template was mixed with a plasmid having a single DNA binding domain. By a Golden Gate reaction, 4 DNA binding domains were linked together (STEP1 plasmid). Thereafter, a vector constructed by In-Fusion cloning (Clontech Laboratories, Inc.) using pcDNA-TAL-NC2 vector (Addgene) as a template was mixed with the STEP1 plasmid. A TALEN plasmid was obtained by the second Golden Gate reaction. The full length sequence of the plasmid is shown in SEQ ID NOs: 1 and 2 (Left_TALEN) and SEQ ID NOs: 3 and 4 (Right_TALEN) of the Sequence Listing.

### 1-2. Construction of Donor Vector for Target Integration (TAL-PITCh Vector):

A plasmid having a modified TALEN sequence in which the first half (first nucleotide sequence) of the spacer of the tyrTALEN target sequence was replaced with the second half (second nucleotide sequence) thereof was constructed (Fig. 1). Inverse PCR was performed with a primer set that adds the above sequence (Xltyr-CMVEGFP-F+Xltyr-CMVEGFP-R; the sequence is shown in Table 1 as described later) using a pCS2/EGFP plasmid with GFP inserted into the ClaI and XbaI sites of pCS2+ as a template. Then, DpnI (New England Biolabs) was added to the PCR reaction solution and the template plasmid was digested. The purified reaction solution was subjected to self ligation, followed by subcloning. A plasmid was prepared from the clone in which accurate insertion was confirmed by sequence analysis and the plasmid was used as a donor vector (The sequence is shown in SEQ ID NOs: 5 and 6 of the Sequence Listing. In SEQ ID NO: 5, the nucleotide sequences 98 to 817 represent an ORF sequence of EGFP. This sequence is inserted into the ClaI/XbaI site of pCS2+. In SEQ ID NO: 5, the nucleotide sequences 1116 to 1167 represent a sequence recognized by the modified TALEN.).

### 1-3. Microinjection into Xenopus Laevis:

On the day preceding the experiment, human pituitary gonadotrophin (ASKA Pharmaceutical Co., Ltd.) was administered to the male Xenopus laevis and the female Xenopus laevis. The administered units were 150 units (for male) and 600 units (for female). On the next day, several drops of sperm suspension was added to the collected eggs and the eggs were artificially inseminated. After about 20 minutes, a 3% cysteine solution was added to allow the fertilized eggs to be dejellied. Then, the resulting eggs were washed several times with 0.1xMMR (ringer solution for amphibians) and transferred into 5% Ficoll/0.3xMMR. The tyrosinase TALEN mRNA mix (Left, Right 250 pg each) and donor vector (100 pg) constructed in the sections 1-1 and 1-2 were co-introduced into the fertilized eggs by the microinjection method (experimental group). As a negative control, only the TALEN mRNA Right (250 pg) and donor vector (100 pg) were co-introduced. Embryos were cultured at 20°C and transferred into 0.1xMMR at the blastula stage to facilitate their development.

### 1-4. Detection of Target Integration:

The embryos (at the tadpole stage) into which the TALEN and vector were co-introduced were observed under a fluorescence stereoscopic microscope and the presence or absence of GFP fluorescence was determined. A genomic DNA for each individual was extracted from the embryos of the control and experimental groups. The introduction of the donor vector into the target site was determined by PCR. The junctions between the genome and the 5'- or 3'-side of the vector were amplified by PCR using the primer set designed at the upstream and downstream of the TALEN target sequence and the vector side. The primer set of tyr-genomic-F and pCS2-R was used for the 5'-side, and the primer set of tyr-genomic-R and pCS2-F was used for the 3'-side (the sequence is shown in Table 1 as described later). After agarose electrophoresis confirmation, a band of the target size was cut out and subcloned into pBluescript SK. The inserted sequence was amplified by colony PCR, followed by analysis by direct sequencing. The sequencing was performed using CEQ-8000 (Beckman Coulter, Inc.).

### Result:

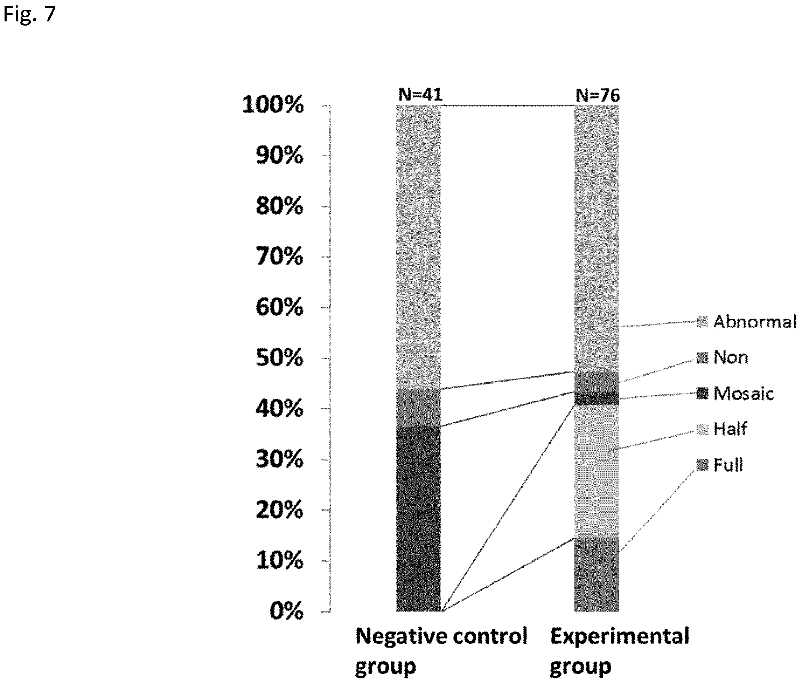

As for the embryos (at the tadpole stage) into which the donor vector was introduced, Figs. 6A and 6B shows phenotypes of the experimental group (TALEN mix + vector-injected embryo) and the negative control group (TALEN R + vector-injected embryo). In the experimental group, the tyr gene was broken and thus an albino phenotype was exhibited in the retinal pigment epithelium and melanophores. Additionally, many individuals generating strong GFP fluorescence throughout the body were observed (Fig. 6B). No albino was observed in the negative control group. Individuals generating mosaic GFP fluorescence were partially observed (Fig. 6A). The ratio of the phenotypes in the experimental group and the negative control group was classified into four groups: Full: the individuals in which GFP fluorescence is observed in the whole body; Half: the individuals in which half of the right or left side has fluorescence; Mosaic: the individuals with mosaic fluorescence; and Non: the individuals in which GFP fluorescence is not observed (Fig. 7). The individuals of Full and Half were not observed in the negative control group, meanwhile, about 20% of the survived individuals exhibited phenotypes of Full and about 50% of the survived individuals exhibited phenotypes of Half in the experimental group.

Subsequently, a genomic DNA was respectively extracted from 5 tadpoles exhibiting phenotypes of Full and 3 individuals of the negative control group observed in Fig. 6, followed by genotyping. In order to confirm the inserted portion on the genome and the junction of the vector, the junctions between the target site and the 5'- or 3'-side of the donor vector were amplified by PCR using the primer set designed at the upstream and downstream of the tyrTALEN target sequence and the vector side (Fig. 8). The PCR products were subjected to electrophoresis and bands having an estimated size were confirmed in the experimental group Nos. 1, 3 and 4 (at the 5'-side) and the experimental group Nos. 2, 3 and 4 (at the 3'-side) (Fig. 8, indicated by arrows). On the other hand, no PCR product was confirmed in the negative control group. Then, in order to examine the sequence of the junctions, the PCR products at the 5'- and 3'-sides detected in Nos. 3 and 4 were subcloned, followed by sequence analysis. As a result, the sequence expected in the case of being joined by MMEJ was confirmed at a ratio of 100% (5/5 clone) in the junction at the 5'-side in No. 3, meanwhile, the sequence expected was confirmed at a ratio of 80% (4/5 clone) in the junction at the 3'-side (Fig. 9A) . The sequence with 10 bases deleted or 3 bases inserted was confirmed in the junction at the 5'-side in No. 4, meanwhile, the sequence expected was confirmed at a ratio of 100% (3/3) in the junction at the 3'-side (Fig. 9B).

The sequences of the primers used in the sections 1-1 to 1-4 are shown in Table 1 below.

**[Table 1]**

| SEQ ID NO: | Primer name | Sequence (from 5' to 3') |
|---|---|---|
| 7 | Xltyr-CMVEGFP-F | |
| 8 | Xltyr-CMVEGFP-R | |
| 9 | tyr-genomic-F | GGAGAGGATGGCCTCTGGAGAGATA |
| 10 | tyr-genomic-R | GGTGGGATGGATTCCTCCCAGAAG |
| 11 | pCS2-F | ATAAGATACATTGATGAGTTTGGAC |
| 12 | pCS2-R | ATGCAGCTGGCACGACAGGTTTCCC |

### Example 2

### Target Integration into HEK293T Cell Using CRISPR/Cas9 System

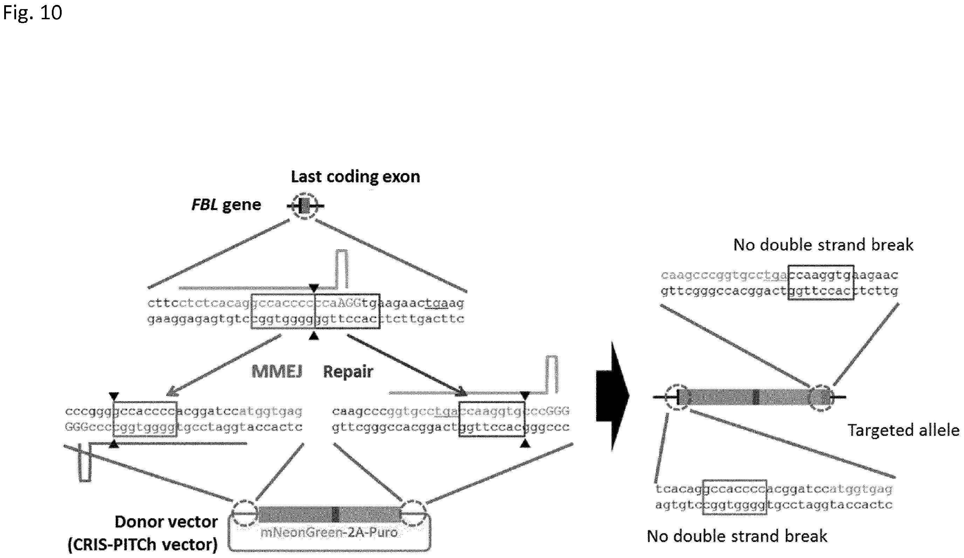

In this example, a fluorescent protein gene expression cassette was introduced (target integration) into the last coding exon of fibrillarin (FBL) gene in a HEK293T cell using the CRISPR/Cas9 system. The outline of this example is illustrated in Fig. 10. Briefly, the vector expressing three types of gRNAs indicated in orange, red and green in Fig. 10 and Cas9 and the donor vector (CRIS-PITCh vector) were co-introduced into the HEK293T cell and the resulting cell was selected by puromycin. Thereafter, DNA sequencing and fluorescent observation were carried out.

### 2-1. Construction of Vector Expressing gRNA and Cas9:

A vector simultaneously expressing three types of gRNAs, and Cas9 was constructed as described in SCIENTIFIC REPORTS 2014 Jun 23; 4: 5400. doi: 10.1038/srep05400. Briefly, the pX330 vector (Addgene; Plasmid 42230) was modified so that a plurality of gRNA expression cassettes could be linked by a Golden Gate reaction. The annealed synthetic oligonucleotides were inserted into three types of modified pX330 vectors. Specifically, oligonucleotides 13 and 14 were annealed to each other to produce a synthetic oligonucleotide for forming a genome cleavage gRNA (indicated in orange in Fig. 10). Further, oligonucleotides 15 and 16 were annealed to each other to produce a synthetic oligonucleotide for forming a genome cleavage gRNA at the 5'-side of the donor vector (indicated in red in Fig. 10). Further, oligonucleotides 17 and 18 were annealed to each other to produce a synthetic oligonucleotide for forming a genome cleavage gRNA at the 3'-side of the donor vector (indicated in green in Fig. 10). Each of the produced synthetic oligonucleotides was inserted into each of the plasmids and then the vectors were integrated by a Golden Gate reaction, and a vector simultaneously expressing three types of gRNAs, and Cas9 was obtained.

### 2-2. Construction of Donor Vector for Target Integration (CRIS-PITCh Vector):

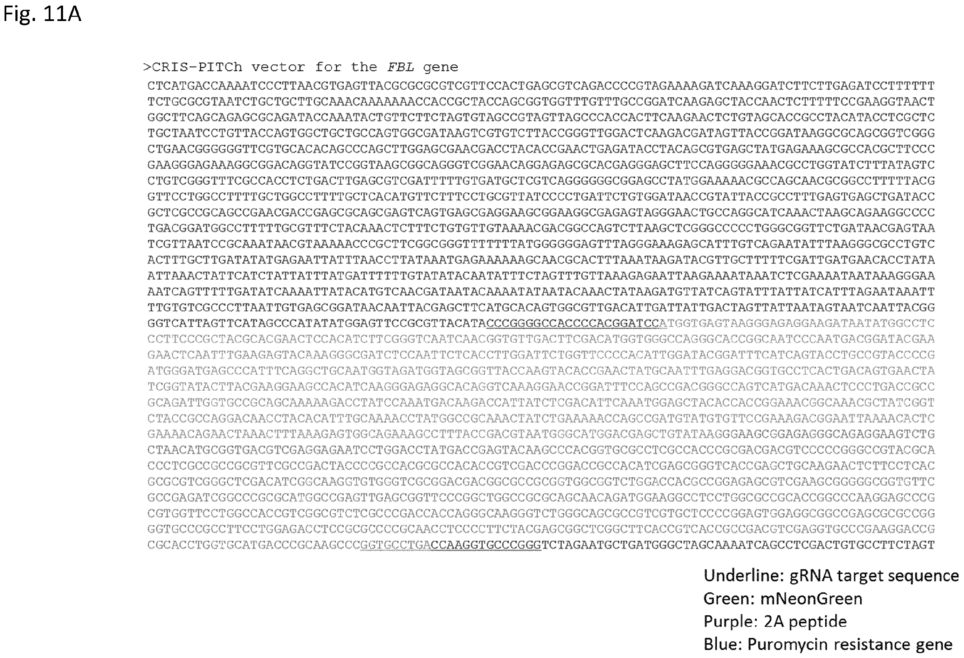

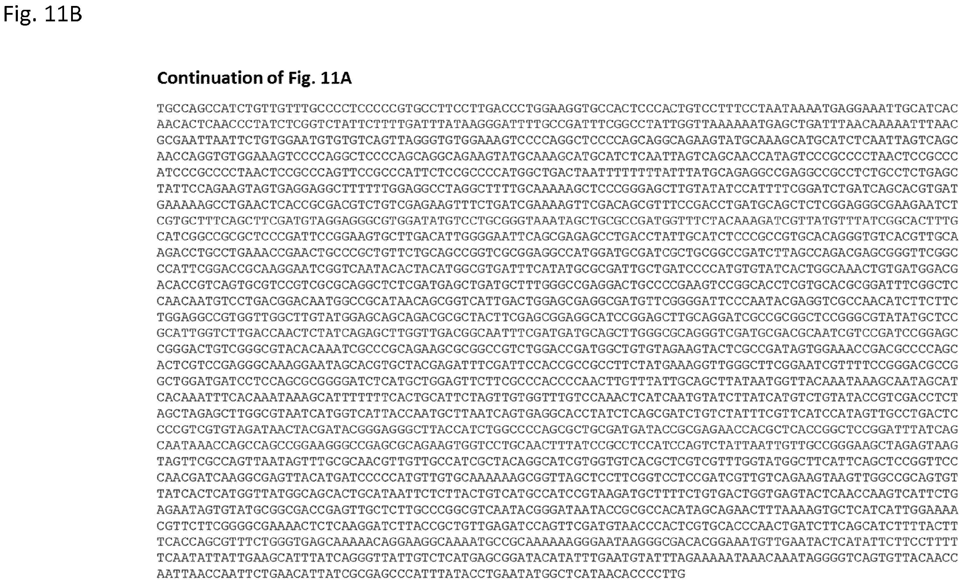

The CRIS-PITCh vector was constructed in the following manner. While a CMV promoter on the vector based on pCMV (Stratagene) was removed, In-Fusion cloning was used to construct a vector such that the gRNA target sequence at the 5'-side, the mNeonGreen coding sequence, the 2A peptide coding sequence, the puromycin resistance gene coding sequence and the gRNA target sequence at the 3'-side were aligned in this order. Figs. 11A and 11B show the full length sequence (SEQ ID NO: 23) of the constructed vector. In Figs. 11A and 11B, the mNeonGreen coding sequence is indicated in green (nucleotides 1566 to 2273 of SEQ ID NO: 23), the 2A peptide coding sequence is indicated in purple (nucleotides 2274 to 2336 of SEQ ID NO: 23), and the puromycin resistance gene coding sequence is indicated in blue (nucleotides 2337 to 2936 of SEQ ID NO: 23). The gRNA target sequences at the 5'- and 3'-sides are underlined.

### 2-3. Introduction into HEK293T Cells:

Introduction into HEK293T cells was performed in the following manner. HEK293T cells were cultured in 10% fetal bovine serum-containing Dulbecco's modified Eagle's medium (DMEM). The cultured cells were seeded at a density of 1 x 10⁵ cells per well on a 6-well plate on the day before the introduction of plasmids. In the introduction of plasmids, 400 ng of a vector expressing a gRNA and Cas9 and 200 ng of a CRIS-PITCh vector were introduced using Lipofectamine LTX (Life Technologies). After the introduction of plasmids, the cells were cultured in a drug-free medium for 3 days and then cultured in a culture medium containing 1 µg/mL of puromycin for 6 days. Thereafter, the cultured cells were single-cell cloned on a 96-well plate by limiting dilution.

### 2-4. Detection of Target Integration:

The HEK293T cell into which the vector expressing a gRNA and Cas9 and the CRIS-PITCh vector were co-introduced was observed using a confocal laser scanning microscope, and the presence or absence of fluorescence was determined. Then, the genomic DNA was extracted from a clone of puromycin resistant cells and the introduction of the donor vector into the target site was confirmed. The junctions between the genome and the 5'- or 3'-side of the vector were amplified by PCR using the primer set designed at the upstream and downstream of the CRISPR target sequence. The primer set of primers 19 and 20 was used for the 5'-side, and the primer set of primers 21 and 22 was used for the 3'-side (the sequence is shown in Table 2 as described later). After agarose electrophoresis confirmation, a band of the target size was cut out and analyzed by direct sequencing. The sequencing was performed using ABI 3130xl Genetic analyzer (Life Technologies).

### Result:

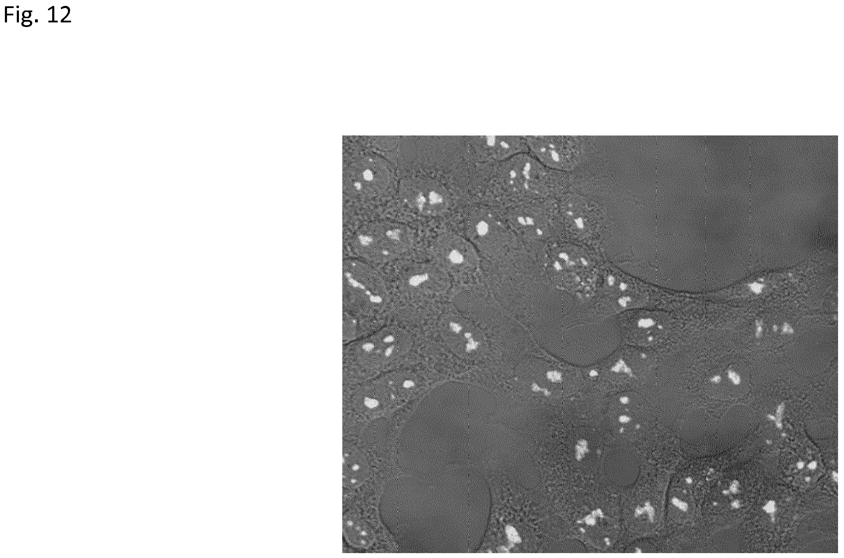

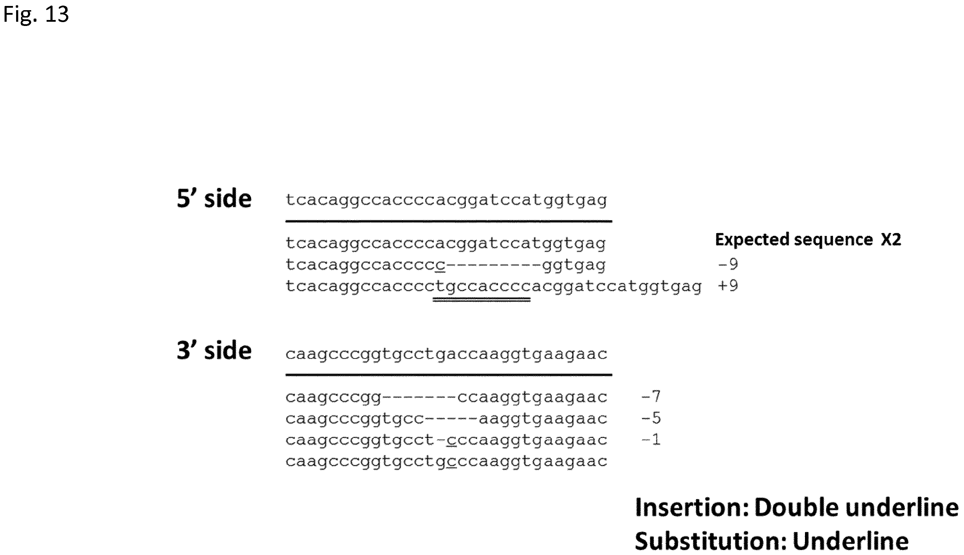

The result observed with the confocal laser scanning microscope is shown in Fig. 12. FBL is a protein specific to nucleoli. Accordingly, in the case where the target integration of the fluorescent protein gene to the FBL gene is successful, the fluorescent protein is localized in the nucleoli. As shown in Fig. 12, a fluorescence image corresponding to the localization pattern (nucleoli) of the FBL protein was obtained. Subsequently, the sequences of the junctions between the genome and the 5'- or 3'-side of the introduced vector were examined. As a result, the sequence expected when the junction at the 5'-side was joined by MMEJ was present at a ratio of 50% (2/4 clone). The remaining two clones had 9 bases deleted or inserted (Fig. 13). The completely expected sequence in the junction at the 3'-side was present at 0% (0/4 clone), but the sequence in which only one base was substituted was present (1 clone). In addition, it was confirmed that one clone had one base deleted, one clone had 5 bases deleted, and one clone had 7 bases deleted (Fig. 13) . Similarly, when the fluorescent protein gene expression cassette was introduced into a β-actin (ACTB) locus of HCT116 cells using the CRISPR/Cas9 system (target integration), the same result as that of the target integration into the HEK293T cell was obtained.

The sequences of the oligonucleotides used in the sections 2-1 to 2-4 are shown in Table 2 below.

**[Table 2]**

| SEQ ID NO: | Name | Sequence (from 5' to 3') |
|---|---|---|
| 13 | Oligonucleotide 13 | CACCGCTCTCACAGGCCACCCCCCA |
| 14 | Oligonucleotide 14 | AAACTGGGGGGTGGCCTGTGAGAGC |
| 15 | Oligonucleotide 15 | CACCGTGGATCCGTGGGGTGGCCCC |
| 16 | Oligonucleotide 16 | AAACGGGGCCACCCCACGGATCCAC |
| 17 | Oligonucleotide 17 | CACCGGTGCCTGACCAAGGTGCCC |
| 18 | Oligonucleotide 18 | AAACGGGCACCTTGGTCAGGCACC |
| 19 | Primer 19 | ACACCAAGACAGACATCTCTGTCCCTTG |
| 20 | Primer 20 | ATCCGTATCCAATGTGGGGPAC |
| 21 | Primer 21 | CCGCAACCTCCCCTTCTACGAG |
| 22 | Primer 22 | TCAGCAGGTCAAGGGGAGGAATG |

## Claims

1. An *in vitro* method for inserting a desired nucleic acid into a predetermined site in a nucleic acid contained in a cell,
wherein the nucleic acid contained in the cell includes a region formed of a first nucleotide sequence, the predetermined site, and a region formed of a second nucleotide sequence in the stated order in a 5'-end to 3'-end direction,
the method comprising:
introducing a vector encoding a nuclease into the cell, wherein the nuclease specifically cleaves a moiety including the region formed of the first nucleotide sequence and the region formed of the second nucleotide sequence included in the cell,
introducing a donor vector into the cell, wherein the donor vector includes a region formed of a first nucleotide sequence, the desired nucleic acid, and a region formed of a second nucleotide sequence in the stated order in a 5'-end to 3'-end direction,
wherein the nuclease acts on the donor vector to produce a nucleic acid fragment including the region formed of the first nucleotide sequence, the desired nucleic acid, and the region formed of the second nucleotide sequence in the stated order in the 5'-end to 3'-end direction, and
wherein the first nucleotide sequence in the nucleic acid contained in the cell and the first nucleotide sequence from the donor vector are joined by microhomology-mediated end joining, and the second nucleotide sequence in the nucleic acid contained in the cell and the second nucleotide sequence from the donor vector are joined by microhomology-mediated end joining, whereby the desired nucleic acid is inserted.

2. The method according to claim 1,
wherein the nuclease includes a first DNA binding domain and a second DNA binding domain,
wherein the region formed of the first nucleotide sequence, the predetermined site and the region formed of the second nucleotide sequence in the nucleic acid contained in the cell are each located between a region formed of a nucleotide sequence recognized by the first DNA binding domain and a region formed of a nucleotide sequence recognized by the second DNA binding domain, and
wherein the region formed of the first nucleotide sequence and the region formed of the second nucleotide sequence in the donor vector are each located between the region formed of the nucleotide sequence recognized by the first DNA binding domain and the region formed of the nucleotide sequence recognized by the second DNA binding domain.

3. The method according to claim 2, wherein the nuclease is a homodimeric nuclease and the donor vector is a circular vector.

4. The method according to claim 1, wherein the nuclease is a Cas9 nuclease.

5. The method according to claim 2, wherein the nuclease is a TALEN.

6. The method according to any one of claims 1 to 5, wherein the first nucleotide sequence is from 3 to 10 bases in length and the second nucleotide sequence is from 3 to 10 bases in length.

7. An *in vitro* method for producing a non-human organism comprising a desired nucleic acid, comprising a step wherein cells are produced by the method according to any preceding claim, and a step wherein the cells produced are differentiated.

## Patentansprüche

1. In-vitro-Verfahren zum Einfügen einer gewünschten Nucleinsäure in eine vorbestimmte Stelle in einer Nucleinsäure, die in einer Zelle enthalten ist;
wobei die in der Zelle enthaltene Nucleinsäure einen Bereich, der aus einer ersten Nucleotidsequenz besteht, die vorbestimmte Stelle und einen Bereich, der aus einer zweiten Nucleotidsequenz besteht, in der genannten Reihenfolge in 5'→3'-Richtung umfasst;
wobei das Verfahren umfasst:
Einführen eines Vektors, der eine Nuclease codiert, in die Zelle, wobei die Nuclease spezifisch eine Struktureinheit spaltet, die den aus der ersten Nucleotidsequenz bestehenden Bereich und den aus der zweiten Nucleotidsequenz bestehenden Bereich, die in der Zelle enthalten sind, umfasst;
Einführen eines Donorvektors in die Zelle, wobei der Donorvektor einen Bereich, der aus einer ersten Nucleotidsequenz besteht, die gewünschte Nucleinsäure und einen Bereich, der aus einer zweiten Nucleotidsequenz besteht, in der genannten Reihenfolge in 5'→3'-Richtung umfasst;
wobei die Nuclease so auf den Donorvektor wirkt, dass ein Nucleinsäurefragment entsteht, das den aus der ersten Nucleotidsequenz bestehenden Bereich, die gewünschte Nucleinsäure und den aus der zweiten Nucleotidsequenz bestehenden Bereich in der genannten Reihenfolge in 5'→3'-Richtung umfasst; und
wobei die erste Nucleotidsequenz in der Nucleinsäure, die in der Zelle enthalten ist, und die erste Nucleotidsequenz aus dem Donorvektor durch mikrohomologievermittelte Endverknüpfung miteinander verknüpft werden und die zweite Nucleotidsequenz in der Nucleinsäure, die in der Zelle enthalten ist, und die zweite Nucleotidsequenz aus dem Donorvektor durch mikrohomologievermittelte Endverknüpfung miteinander verknüpft werden, wodurch die gewünschte Nucleinsäure eingefügt wird.

2. Verfahren gemäß Anspruch 1,
wobei die Nuclease eine erste DNA-Bindungsdomäne und eine zweite DNA-Bindungsdomäne umfasst;
wobei sich der Bereich, der aus der ersten Nucleotidsequenz besteht, die vorbestimmte Stelle und der Bereich, der aus der zweiten Nucleotidsequenz besteht, in der Nucleinsäure, die in der Zelle enthalten ist, jeweils zwischen einem Bereich, der aus einer Nucleotidsequenz besteht, die von der ersten DNA-Bindungsdomäne erkannt wird, und einem Bereich, der aus einer Nucleotidsequenz besteht, die von der zweiten DNA-Bindungsdomäne erkannt wird, befinden; und
wobei sich der Bereich, der aus der ersten Nucleotidsequenz besteht, und der Bereich, der aus der zweiten Nucleotidsequenz besteht, in dem Donorvektor jeweils zwischen dem Bereich, der aus der Nucleotidsequenz besteht, die von der ersten DNA-Bindungsdomäne erkannt wird, und dem Bereich, der aus der Nucleotidsequenz besteht, die von der zweiten DNA-Bindungsdomäne erkannt wird, befinden.

3. Verfahren gemäß Anspruch 2, wobei die Nuclease eine homodimere Nuclease ist und der Donorvektor ein zirkulärer Vektor ist.

4. Verfahren gemäß Anspruch 1, wobei die Nuclease eine Cas9-Nuclease ist.

5. Verfahren gemäß Anspruch 2, wobei die Nuclease eine TALEN ist.

6. Verfahren gemäß einem der Ansprüche 1 bis 5, wobei die erste Nucleotidsequenz eine Länge von 3 bis 10 Basen aufweist und die zweite Nucleotidsequenz eine Länge von 3 bis 10 Basen aufweist.

7. In-vitro-Verfahren zur Herstellung eines nichthumanen Organismus, der eine gewünschte Nucleinsäure umfasst, umfassend einen Schritt, bei dem Zellen nach dem Verfahren gemäß einem der vorstehenden Ansprüche produziert werden, und einen Schritt, bei dem die produzierten Zellen differenziert werden.

## Revendications

1. Procédé *in vitro* pour insérer un acide nucléique souhaité dans un site prédéterminé dans un acide nucléique contenu dans une cellule,
où l'acide nucléique contenu dans la cellule inclut une région formée d'une première séquence nucléotidique, le site prédéterminé et une région formée d'une seconde séquence nucléotidique dans l'ordre indiqué dans une direction extrémité 5' à extrémité 3',
le procédé comprenant:
l'introduction d'un vecteur codant une nucléase dans la cellule, où la nucléase clive spécifiquement une entité incluant la région formée de la première séquence nucléotidique et la région formée de la seconde séquence nucléotidique incluses dans la cellule,
l'introduction d'un vecteur donneur dans la cellule, où le vecteur donneur inclut une région formée d'une première séquence nucléotidique, l'acide nucléique souhaité, et une région formée d'une seconde séquence nucléotidique dans l'ordre indiqué dans une direction extrémité 5' à extrémité 3',
où la nucléase agit sur le vecteur donneur pour produire un fragment d'acide nucléique incluant la région formée de la première séquence nucléotidique, l'acide nucléique souhaité et la région formée de la seconde séquence nucléotidique dans l'ordre indiqué dans la direction extrémité 5' à extrémité 3', et
où la première séquence nucléotidique de l'acide nucléique contenu dans la cellule et la première séquence nucléotidique provenant du vecteur donneur sont jointes par jonction d'extrémités médiée par microhomologie, et la seconde séquence nucléotidique dans l'acide nucléique contenu dans la cellule et la seconde séquence nucléotidique provenant du vecteur donneur sont jointes par jonction d'extrémités médiée par microhomologie, de sorte que l'acide nucléique souhaité est inséré.

2. Procédé selon la revendication 1,
où la nucléase inclut un premier domaine de liaison à l'ADN et un second domaine de liaison à l'ADN,
où la région formée de la première séquence nucléotidique, le site prédéterminé et la région formée de la seconde séquence nucléotidique dans l'acide nucléique contenu dans la cellule sont situés chacun entre une région formée d'une séquence nucléotidique reconnue par le premier domaine de liaison à l'ADN et une région formée d'une séquence nucléotidique reconnue par le second domaine de liaison à l'ADN, et
où la région formée de la première séquence nucléotidique et la région formée de la seconde séquence nucléotidique dans le vecteur donneur sont situées chacune entre la région formée de la séquence nucléotidique reconnue par le premier domaine de liaison à l'ADN et la région formée de la séquence nucléotidique reconnue par le second domaine de liaison à l'ADN.

3. Procédé selon la revendication 2, où la nucléase est une nucléase homodimère et le vecteur donneur est un vecteur circulaire.

4. Procédé selon la revendication 1, où la nucléase est une nucléase Cas9.

5. Procédé selon la revendication 2, où la nucléase est une TALEN.

6. Procédé selon l'une quelconque des revendications 1 à 5, où la première séquence nucléotidique a une longueur de 3 à 10 bases et la seconde séquence nucléotidique a une longueur de 3 à 10 bases.

7. Procédé *in vitro* pour produire un organisme non humain comprenant un acide nucléique souhaité, comprenant une étape où des cellules sont produites par le procédé par étapes selon l'une quelconque des revendications précédentes, et une étape où les cellules produites sont différenciées.
